# EUROPÄISCHE PATENTSCHRIFT

(11) **EP 1 497 262 B1**
(45) Veröffentlichungstag und Bekanntmachung des Hinweises auf die Patenterteilung: **04.08.2010**
(21) Anmeldenummer: 03712111.8
(22) Anmeldetag: 28.03.2003
(51) Int. Cl.: C07C 275/54, C07D 295/12, A61K 31/17, A61P 7/12

(54) **ACYL-4-CARBOXY-PHENYL-HARNSTOFFDERIVATE, VERFAHREN ZU DEREN HERSTELLUNG UND DEREN VERWENDUNG**
ACYL-4-CARBOXYPHENYLUREA DERIVATIVES, METHOD FOR PRODUCTION AND USE THEREOF
DERIVES D'ACYL-4-CARBOXYPHENYLUREE, PROCEDES DE PRODUCTION DE CES DERIVES ET LEUR UTILISATION

(30) Priorität: 11.04.2002 DE 10215907
(43) Veröffentlichungstag der Anmeldung: 19.01.2005
(73) Patentinhaber: Sanofi-Aventis Deutschland GmbH, 65929 Frankfurt am Main (DE)
(72) Erfinder: DEFOSSA, Elisabeth, 65510 Idstein (DE); KADEREIT, Dieter, 65779 Kelkheim (DE); SCHOENAFINGER, Karl, 63755 Alzenau (DE); KLABUNDE, Thomas, 65929 Frankfurt (DE); BURGER, Hans-Joerg, Morristown, NJ 07960 (US); HERLING, Andreas, 65520 Bad Camberg (DE); WENDT, Karl-Urlich, 60433 Frankfurt (DE); VON ROEDERN, Erich, 65795 Hattersheim (DE); ENHSEN, Alfons, 64572 Büttelborn (DE); RIEKE-ZAPP, Joerg, 60325 Frankfurt (DE)
(86) Internationale Anmeldenummer: PCT/EP2003/003251
(87) Internationale Veröffentlichungsnummer: WO 2003/084922

(56) Entgegenhaltungen:
- EP-A- 0 136 745
- EP-A- 0 193 249
- EP-A- 0 298 314
- WO-A-01/94300
- WO-A-02/081463
- WO-A-02/096864
- WO-A-03/084923
- WO-A1-03/084923

## Beschreibung

Die Erfindung betrifft Acyl-4-carboxyphenyl-harnstoffderivate sowie deren physiologisch verträgliche Salze und physiologisch funktionelle Derivate.

In EP 0 193 249 (Duphar) werden Acyl-carboxyphenyl-harnstoffderivate mit Antitumoraktivität beschrieben.

Der Erfindung lag die Aufgabe zugrunde, Verbindungen zur Verfügung zu stellen, mit denen eine Prävention und Behandlung von Diabetes Typ 2 möglich ist. Insbesonders lag die Aufgabe darin, dass neue Verbindungen mit einer deutlich verbesserten Wirkung gegenüber den aus EP 0 193 249 bekannten Verbindungen zur Verfügung gestellt werden.

Die Erfindung betrifft daher Verbindungen der Formel I, 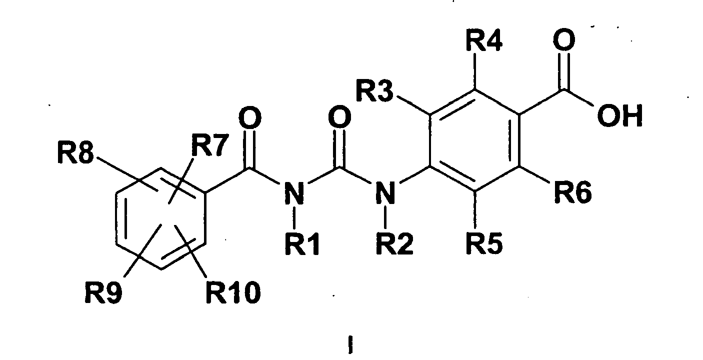 worin bedeuten
- R7, R8, R9, R10: unabhängig von einander H, F, Cl, Br, OH, NO₂, CN, O-(C₁- C₆)-Alkyl, O-(C₂-C₆)-Alkenyl, O-(C₂-C₆)-Alkinyl, O-SO₂-(C₁-C₄)-Alkyl, (C₁- C₆)-Alkyl, (C₂-C₆)-Alkenyl, (C₂-C₆)-Alkinyl, wobei Alkyl, Alkenyl und Alkinyl ein oder mehrfach durch F, Cl oder Br substituiert sein können;
- R1, R2: unabhängig voneinander H, (C₁-C₆)-Alkyl, wobei Alkyl mit OH, O-(C₁- C₄)-Alkyl, NH₂, NH(C₁-C₄)-Alkyl, N[(C₁-C₆)-Alkyl]₂ substituiert sein kann, O-(C₁-C₆)-Alkyl, CO-(C₁-C₆)-Alkyl, COO-(C₁-C₆)-Alkyl, (C₁-C₆)-Alkylen- COOH oder (C₁-C₆)-Alkylen-COO-(C₁-C₆)-alkyl,
- R3: H, F, Cl, Br, NO₂, CN, O-R11, O-Phenyl, S-R11, COOR11, N(R12)(R13), (C₁-C₆)-Alkyl, (C₂-C₆)-Alkenyl, (C₂-C₆)-Alkinyl, (C₃-C₇)- Cycloalkyl oder (C₃-C₇)-Cycloalkyl-(C₁-C₄)-alkylen, wobei Alkyl, Cycloalkyl und Alkinyl ein oder mehrfach mit F, Cl, Br, OR11, COOR11 oder N(R16)(R17) substituiert sein können;
- R4: H, F, Cl, Br, NO₂, CN, O-R11, O-Phenyl, S-R11, COOR11, N(R12)(R13), (C₁-C₆)-Alkyl, (C₂-C₆)-Alkenyl, (C₂-C₆)-Alkinyl, (C₃-C₇)- Cycloalkyl oder (C₃-C₇)-Cycloalkyl-(C₁-C₄)-alkylen, wobei Alkyl, Cycloalkyl und Alkinyl ein oder mehrfach mit F, Cl, Br, OR11, COOR11 oder N(R16)(R17) substituiert sein können;
- R5: H, F, Cl, Br, NO₂, CN, O-R11, O-Phenyl, S-R11, COOR11, N(R12)(R13), (C₁-C₆)-Alkyl, (C₂-C₆)-Alkenyl, (C₂-C₆)-Alkinyl, (C₃-C₇)- Cycloalkyl oder (C₃-C₇)-Cycloalkyl-(C₁-C₄)-alkylen, wobei Alkyl, Cycloalkyl und Alkinyl ein oder mehrfach mit F, Cl, Br, OR11, COOR11 oder N(R16)(R17) substituiert sein können;
- R6: H, F, Cl, Br, NO₂, CN, O-R11, O-Phenyl, S-R11, COOR11, N(R12)(R13), (C₁-C₆)-Alkyl, (C₂-C₆)-Alkenyl, (C₂-C₆)-Alkinyl, (C₃-C₇)- Cycloalkyl oder (C₃-C₇)-Cycloalkyl-(C₁-C₄)-alkylen, wobei Alkyl, Cycloalkyl und Alkinyl ein oder mehrfach mit F, Cl, Br, OR11, COOR11 oder N(R16)(R17) substituiert sein können;
- R11: H, (C₁-C₈)-Alkyl, (C₂-C₈)-Alkenyl oder (C₂-C₈)-Alkinyl, wobei Alkyl, Alkenyl und Alkinyl ein oder mehrfach mit F, Cl, Br, OH oder O-(C₁-C₄)- Alkyl substituiert sein können;
- R12, R13: unabhängig voneinander H, (C₁-C₈)-Alkyl, (C₂-C₈)-Alkenyl, (C₂-C₈)- Alkinyl, (C₃-C₇)-Cycloalkyl, (C₃-C₇)-Cycloalkyl-(C₁-C₄)-alkylen, COO-(C₁- C₄)-Alkyl, COO-(C₂-C₄)-Alkenyl, Phenyl oder SO₂-Phenyl, wobei der Phenylring bis zu zweifach mit F, Cl, CN, OH, (C₁-C₆)-Alkyl, O-(C₁-C₆)- Alkyl, CF₃, OCF₃, COOH, COO-(C₁-C₆)-Alkyl oder CONH₂ substituiert sein kann;
- oder R12 und R13: bilden gemeinsam mit dem Stickstoffatom an das sie gebunden sind einen 3-7 gliedrigen, gesättigten heterozyklischen Ring, der bis zu 2 weitere Heteroatome aus der Gruppe N, O oder S enthalten kann und wobei der heterozyklische Ring bis zu vierfach mit F, Cl, Br, OH, Oxo, (C₁-C₄)-Alkyl oder N(R14)(R15) substituiert sein kann;
- R14, R15: unabhängig voneinander H, (C₁-C₈)-Alkyl, (C₂-C₈)-Alkenyl, (C₂-C₈)- Alkinyl, (C₃-C₇)-Cycloalkyl; (C₃-C₇)-Cycloalkyl-(C₁-C₄)-alkylen, COO-(C₁- C₄)-Alkyl, COO-(C₂-C₄)-Alkenyl, Phenyl oder SO₂-Phenyl, wobei der Phenylring bis zu zweifach mit F, Cl, CN, OH, (C₁-C₆)-Alkyl, O-(C₁-C₆)- Alkyl, CF₃, OCF₃, COOH, COO(C₁-C₆)-Alkyl oder CONH₂ substituiert sein kann;
- R16, R17: unabhängig voneinander H, (C₁-C₈)-Alkyl, (C₂-C₈)-Alkenyl, (C₂-C₈)- Alkinyl, (C₃-C₇)-Cycloalkyl, (C₃-C₇)-Cycloalkyl-(C₁-C₄)-alkylen, COO-(C₁- C₄)-Alkyl, COO-(C₂-C₄)-Alkenyl, Phenyl oder SO₂-Phenyl, wobei der Phenylring bis zu zweifach mit F, Cl, CN, OH, (C₁-C₆)-Alkyl, O-(C₁-C₆)- Alkyl, CF₃, OCF₃, COOH, COO-(C₁-C₆)-Alkyl oder CONH₂ substituiert sein kann;
- oder R16 und R17: bilden gemeinsam mit dem Stickstoffatom an das sie gebunden sind einen 3-7 gliedrigen, gesättigten heterozyklischen Ring, der bis zu 2 weitere Heteroatome aus der Gruppe N, O oder S enthalten kann und wobei der heterozyklische Ring bis zu vierfach mit F, Cl, Br, OH, Oxo, (C₁-C₄)-Alkyl oder N(R14)(R15) substituiert sein kann;
wobei immer mindestens einer der Reste R3, R7, R8, R9 und R10 ungleich Wasserstoff ist und
wobei die Verbindung 4-[3-(2,4-Dichloro-benzoyl)-ureido]-3-methoxy-benzoesäure ausgeschlossen ist;
sowie deren physiologisch verträgliche Salze.

Bevorzugt sind Verbindungen der Formel I worin ein oder mehrere Reste folgende Bedeutung haben:
- R7, R8, R9, R10: unabhängig von einander H, F, Cl, Br, OH, NO₂, CN, (C₁-C₆)- Alkyl oder O-(C₁-C₆)-Alkyl, wobei Alkyl ein oder mehrfach durch F substituiert sein kann;
- R1, R2: H;
- R3: H, F, Cl, Br, NO₂, CN, O-R11, O-Phenyl, S-R11, COOR11, N(R12)(R13), (C₁-C₆)-Alkyl, (C₂-C₆)-Alkenyl, (C₂-C₆)-Alkinyl, (C₃-C₇)- Cycloalkyl oder (C₃-C₇)-Cycloalkyl-(C₁-C₄)-alkylen, wobei Alkyl, Cycloalkyl und Alkinyl ein oder mehrfach mit F, Cl, Br, OR11 oder COOR11 substituiert sein können;
- R4: H, F, Cl, NO₂, O-R11, N(R12)(R13) oder (C₁-C₆)-Alkyl, wobei Alkyl ein oder mehrfach mit F substituiert sein kann;
- R5: H, F, Cl, NO₂, O-R11, N(R12)(R13) oder (C₁-C₆)-Alkyl, wobei Alkyl ein oder mehrfach mit F substituiert sein kann;
- R6: H, F, Cl, NO₂, O-R11, N(R12)(R13) oder (C₁-C₆)-Alkyl, wobei Alkyl ein oder mehrfach mit F substituiert sein kann;
- R11: H, (C₁-C₈)-Alkyl, (C₁-C₈)-Alkylen-O-(C₁-C₈)-Alkyl oder (C₁-C₈)-Alkyl-OH, wobei Alkyl ein oder mehrfach durch F substituiert sein kann;
- R12, R13: unabhängig voneinander H oder (C₁-C₈)-Alkyl;

oder R12 und R13 bilden gemeinsam mit dem Stickstoffatom an das sie gebunden sind einen 3-7 gliedrigen, gesättigten heterozyklischen Ring, der bis zu 2 weitere Heteroatome aus der Gruppe N, O oder S enthalten kann und wobei der heterozyklische Ring bis zu vierfach mit F, Cl, Br, OH, Oxo, (C₁-C₄)-Alkyl oder N(R14)(R15) substituiert sein kann;
   - R14, R15: unabhängig voneinander H, (C₁-C₈)-Alkyl, (C₂-C₈)-Alkenyl, (C₂-C₈)- Alkinyl, (C₃-C₇)-Cycloalkyl, (C₃-C₇)-Cycloalkyl-(C₁-C₄)-alkylen, COO-(C₁- C₄)-Alkyl, COO-(C₂-C₄)-Alkenyl, Phenyl oder SO₂-Phenyl, wobei der Phenylring bis zu zweifach mit F, Ci, CN, OH, (C₁-C₆)-Alkyl, O-(C₁-C₆)- Alkyl, CF₃, OCF₃, COOH, COO(C₁-C₆)-Alkyl oder CONH₂ substituiert sein kann;
   wobei immer mindestens einer der Reste R3, R7, R8, R9 und R10 ungleich Wasserstoff ist und
   wobei die Verbindung 4-[3-(2,4-Dichloro-benzoyl)-ureido]-3-methoxy-benzoesäure ausgeschlossen ist;
   sowie deren physiologisch verträgliche Salze.
   Ganz besonders bevorzugt sind Verbindungen der Formel I worin ein oder mehrere Reste folgende Bedeutung haben:
   - R7, R8, R9, R10: unabhängig von einander H, F, Cl, Br, CH₃ oder CF₃;
   - R1, R2, R5: H;
   - R3: H, F, Cl, NO₂, O-R11, N(R12)(R13) oder (C₁-C₆)-Alkyl, wobei Alkyl ein oder mehrfach mit F substituiert sein kann;
   - R4: H, F, Cl, N0₂, O-R11, N(R12)(R13) oder (C₁-C₆)-Alkyl, wobei Alkyl ein oder mehrfach mit F substituiert sein kann;
   - R6: H, F, Cl, NO₂, O-R11, N(R12)(R13) oder (C₁-C₆)-Alkyl, wobei Alkyl ein oder mehrfach mit F substituiert sein kann;
   - R11: H oder (C₁-C₈)-Alkyl, wobei Alkyl ein oder mehrfach mit F substituiert sein kann,
   - R12, R13: H oder (C₁-C₈)-Alkyl;

oder die beiden Reste R12 und R13 bilden mit dem Stickstoffatom an das sie
   gebunden sind einen 5-6 gliedrigen, gesättigten heterozyklischen Ring, der ein weiteres Sauerstoffatom enthalten kann;
   wobei immer mindestens einer der Reste R3, R7, R8, R9 und R10 ungleich Wasserstoff ist und
   wobei die Verbindung 4-[3-(2,4-Dichloro-benzoyl)-ureido]-3-methoxy-benzoesäure ausgeschlossen ist;
   sowie deren physiologisch verträgliche Salze.
   Weiter ganz besonders bevorzugt sind Verbindungen der Formel I worin ein oder mehrere Reste folgende Bedeutung haben:
   - R7, R8, R9, R10: unabhängig von einander H, F, Cl, Br, CH₃ oder CF₃;
   - R1, R2, R4, R5, R6: H;

   - R3: H, F, Cl, NO₂, O-R11, N(R12)(R13) oder (C₁-C₆)-Alkyl, wobei Alkyl ein oder mehrfach mit F substituiert sein kann;
   - R11: H oder (C₁-C₈)-Alkyl, wobei Alkyl ein oder mehrfach mit F substituiert sein kann,
   - R12, R13: H oder (C₁-C₈)-Alkyl;
oder die beiden Reste R12 und R13 bilden mit dem Stickstoffatom an das sie gebunden sind einen 5-6 gliedrigen, gesättigten heterozyklischen Ring, der ein weiteres Sauerstoffatom enthalten kann;
wobei immer mindestens einer der Reste R3, R7, R8, R9 und R10 ungleich Wasserstoff ist und
wobei die Verbindung 4-[3-(2,4-Dichloro-benzoyl)-ureido]-3-methoxy-benzoesäure ausgeschlossen ist;
sowie deren physiologisch verträgliche Salze

Weiterhin bevorzugt sind Verbindungen der Formel I, worin R3 ungleich H ist.

Können Reste oder Substituenten mehrfach in den Verbindungen der Formel I auftreten, wie zum Beispiel -O-R11, so können sie alle unabhängig voneinander die angegebenen Bedeutungen haben und gleich oder verschieden sein.

Die Erfindung bezieht sich auf Verbindungen der Formel I, in Form ihrer Racemate, racemischen Mischungen und reinen Enantiomere sowie auf ihre Diastereomere und Mischungen davon.

Die Alkylreste in den Substituenten R1, R2, R3, R4, R5, R6, R7, R8, R9, R10, R11 R12, R13, R14, R15, R16 und R17 können sowohl geradkettig wie verzweigt sein. Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin oder Ethylendiamin.

Besonders bevorzugt sind die Trometamolsalze (im folgenden auch TRIS, Trishydroxymethyl-methylamin genannt) der Verbindungen der Formel I. Sie zeigen eine höhere Bioverfügbarkeit, als die entsprechenden freien Säuren.

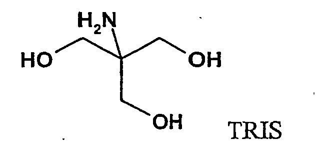

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze, Solvate und physiologisch funktionellen Derivate wie hierin beschrieben.

Die Verbindung(en) der Formel (I) können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Als weitere Wirkstoffe für die Kombinationspräparate sind geeignet: Alle Antidiabetika, die in der Roten Liste 2001, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.
Antidiabetika umfassen Insulin und Insulinderivate, wie z.B. Lantus^{®} (siehe www.lantus.com) oder HMR 1964, schnell wirkende Insuline (siehe US 6,221,633), GLP-1-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulphonylfharnstoffe, Biguanidine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, GI 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, oder wie in PCT/US 11833, PCT/US 11490, DE10142734.4 beschrieben verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide, BMS-201038, R-103757, verabreicht.

Bei einer Ausführungsform der Erfindung werden die Verbindungen der Formel I in Kombination mit einem Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel I in Kombination mit einem CETP-Inhibitor, wie z.B. JTT-705 , verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht. Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen-Synthetase Inhibitor, wie z.B. BMS-188494, verabreicht.

Bei einer Ausführungsform der Erfindung werden die Verbindungen der Formel I in Kombination mit einem Lipoprotein(a) antagonist, wie z.B. CI-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.

Bei einer Ausführungsform werden die Verbindungen der Formel I in Kombination mit einem Sulphonylharnstoff, wie z.B. Tolbutamid, Glibenclamid, Glipizid oder Glimepirid verabreicht.

Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]-phenyl]methyl]-2,4-thiazolidindion, verabreicht.

Bei einer Ausführungsform werden die Verbindungen der Formel I in Kombination mit einem α-Glukosidase-Inhibitor, wie z.B. Miglitol oder Acarbose, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform werden die Verbindungen der Formel I in Kombination mit mehr als einer der vorstehend genannten Verbindungen, z.B. in Kombination mit einem Sulphönylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäure {4-[(4-amino-quinazolin-2-ylamino)-methyl]-cyclohexylmethyl}- amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahyd ro-pyrazolo[4,3-c]pyridin-5-yl)-1-(4-chloro-phenyl)-2-oxo-ethyl]-amid; (WO 01/91752)) , Orexin-Antagonisten (z.B. 1-(2-Methyl-benzoxazol-6-yl)-3-[1,5]naphthyridin-4-yl-harnstoff; hydrochloride (SB-334867-A)), H3-Agonisten (3-Cyclohexyl-1-(4,4-dimethyl-1,4,6,7-tetrahydro-imidazo[4,5-c]pyridin-5-yl)-propan-1- on Oxalsäuresalz (WO 00 / 63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1 H-indol-6-yloxy)-ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7- dimethyl-indol-1-yl}-acetic acid Trifluoressigsäuresalz (WO 99/15525)); Serotonin-Wiederaufnahme-Inhibitoren (z.B. Dexfenflurämine), gemischte Serotonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isoquinoline-2-carboxylic acid tert-butyl ester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881),

DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.

Bei einer Ausführungsform ist der weitere Wirkstoff Fenfluramin oder Dexfenfluramin. Bei noch einer Ausführungsform ist der weitere Wirkstoff Sibutramin.

Bei einer Ausführungsform ist der weitere Wirkstoff Orlistat.

Bei einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird. 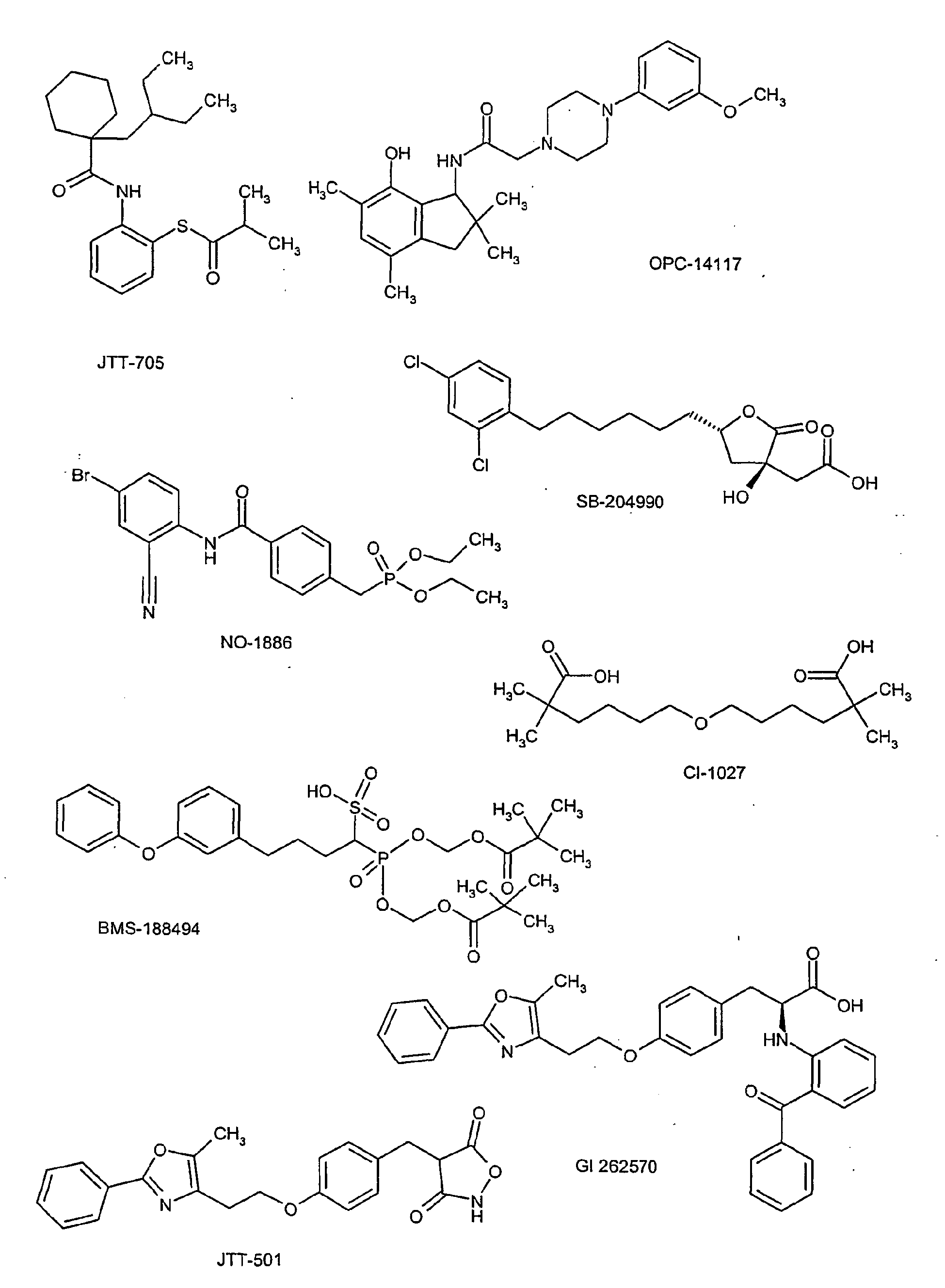 Die nachfolgend aufgeführten Beispiele dienen zur Erläuterung der Erfindung, ohne diese jedoch einzuschränken.

**Tabelle 1: Beispiele der Formel I**

| | | | | | | | | | | |
|---|---|---|---|---|---|---|---|---|---|---|
| 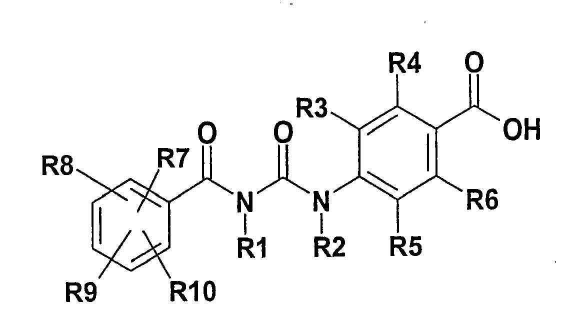 | | | | | | | | | | |

| **Bsp.** | **R7, R8, R9, R10** | **R1** | **R2** | **R3** | **R4** | **R5** | **R6** | **Salz** | **MS**** | **Smp** |
|---|---|---|---|---|---|---|---|---|---|---|
| 1 | 2-Cl, H, H, H | H | H | NO₂ | H | H | H | - | ok | 240* |
| 2 | 2-Cl, 4-Cl, H, H | H | H | NO₂ | H | H | H | - | ok | |
| 3 | 2-Cl, 4-Cl, H, H | H | H | NO₂ | H | H | H | TRIS | ok | |
| 4 | 2-Cl, H, H, H | H | H | OH | H | H | H | - | ok | |
| 5 | 2-Cl, 4-Cl, H, H | H | H | OH | H | H | H | - | ok | |
| 6 | 2-Cl, H, H, H | H | H | Cl | H | H | H | - | ok | |
| 7 | 2-Cl, H, H, H | H | H | OCH₃ | H | H | H | - | ok | |
| 8 | 2-Cl, 4-Cl, H, H | H | H | OCH₃ | H | H | H | - | ok | |
| 9 | 2-Cl, 4-Cl, H, H | H | H | Cl | H | H | H | - | ok | |
| 10 | 2-Cl, 5-Cl, H, H | H | H | Cl | H | H | H | - | ok | |
| 11 | 2-Cl, 5-Cl, H, H | H | H | OCH₃ | H | H | H | - | ok | |
| 12 | 2-F, H, H, H | H | H | OCH₃ | H | H | H | - | ok | |
| 13 | 3-F, H, H, H | H | H | OCH₃ | H | H | H | - | ok | |
| 14 | 3-Cl, H, H, H | H | H | OCH₃ | H | H | H | - | ok | |
| 15 | 2-Cl, 5-CH₃, H, H | H | H | Cl | H | H | H | - | ok | |
| 16 | 2-Cl, 5-CH₃, H, H | H | H | OCH₃ | H | H | H | - | ok | |
| 17 | 4-F, H, H, H | H | H | OCH₃ | H | H | H | - | ok | |
| 18 | 3-Cl, 4-Cl, H, H | H | H | OCH₃ | H | H | H | - | ok | |
| 19 | 2-CH₃, H, H, H | H | H | Cl | H | H | H | - | ok | |
| 20 | 2-F, H, H, H | H | H | Cl | H | H | H | - | ok | |
| 21 | 3-F, H, H, H | H | H | Cl | H | H | H | - | ok | |
| 22 | 3-Cl, H, H, H | H | H | Cl | H | H | H | - | ok | |
| 23 | 3-Cl, 4-Cl, H, H | H | H | Cl | H | H | H | - | ok | |
| 24 | 2-CH₃, H, H, H | H | H | OCH₃ | H | H | H | - | ok | |
| 25 | 2-CH₃, 4-CH₃, H, H | H | H | Cl | H | H | H | - | ok | |
| 26 | 2-CH₃, 4-CH₃, H, H | H | H | OCH₃ | H | H | H | - | ok | |
| 27 | 2-F, 4-Cl, H, H | H | H | OCH₃ | H | H | H | - | ok | |
| 28 | 2-F, 4-Cl, H, H | H | H | Cl | H | H | H | - | ok | |
| 29 | 2-F, 4-F, H, H | H | H | OCH₃ | H | H | H | - | ok | |
| 30 | 2-F, 4-F, H, H | H | H | Cl | H | H | H | - | ok | |
| 31 | 2-Cl, 4-F, H, H | H | H | OCH₃ | H | H | H | - | ok | |
| 32 | 2-Cl, 4-F, H, H | H | H | Cl | H | H | H | - | ok | |
| 33 | 2-Cl, 4-F, H, H | H | H | Cl | H | H | H | TRIS | ok | |
| 34 | 2-F, H, H, H | H | H | H | H | H | H | - | ok | |
| 35 | 2-Cl, H, H, H | H | H | H | H | H | OH | - | ok | |
| 36 | 2-Cl, 4-Cl, H, H | H | H | H | H | H | OH | - | ok | |
| 37 | 2-Cl, 4-F, H, H | H | H | H | H | H | OH | - | ok | |
| 38 | 2-Cl, 4-Cl, H, H | H | H | Cl | H | H | OH | - | ok | |
| 39 | 2-Cl, 4-F, H, H | H | H | Cl | H | H | OH | - | ok | |
| 40 | 2-Cl, 5-Br, H, H | H | H | OCH₃ | H | H | H | - | ok | 251 |
| 41 | 2-Cl, 4-F, 5-F, H | H | H | OCH₃ | H | H | H | - | ok | 268 |
| 42 | 2-Cl, 4-F, 5-F, H | H | H | OCH₃ | H | H | H | TRIS | ok | |
| 43 | 2-Cl, 4-Cl, H, H | H | H | OCF₃ | H | H | H | - | ok | |
| 44 | 2-Cl, 4-F, H, H | H | H | OCF₃ | H | H | H | - | ok | |
| 45 | 2-F, 6-Cl, H, H | H | H | OCH₃ | H | H | H | - | ok | |
| 46 | 2-CH₃, 6-CH₃, H, H | H | H | OCH₃ | H | H | H | - | ok | |
| 47 | 2-Cl, 4-F, 5-F, H | H | H | Cl | H | H | H | - | ok | 262 |
| 48 | 2-Cl, 4-F, 5-F, H | H | H | OCF₃ | H | H | H | - | ok | 236 |
| 49 | 2-Cl, 4-F, 5-F, H | H | H | OCF₃ | H | H | H | TRIS | ok | 176 |
| 50 | 2-Cl, 4-Cl, 5-F, H | H | H | OCH₃ | H | H | H | - | ok | 261 |
| 51 | 2-Cl, 5-F, H, H | H | H | OCH₃ | H | H | H | - | ok | 273 |
| 52 | 2-Cl, 4-F, H, H | H | H | H | H | H | NH₂ | - | ok | 222 |
| 53 | 2-Cl, 4-F, H, H | H | H | CH₃ | H | H | H | - | ok | |
| 54 | 2-Cl, 4-Cl, H, H | H | H | CH₃ | H | H | H | - | ok | |
| 55 | 2-Cl, 4-F, 5-F, H | H | H | CF₃ | H | H | H | - | ok | |
| 56 | 2-Cl, 4-F, H, H | H | H | OCH₃ | H | H | OH | - | ok | 256 |
| 57 | 2-Cl, 4-F, 5-F, H | H | H | OCH₃ | H | H | OH | - | ok | 250 |
| 58 | 2-Br, H, H, H | H | H | OCH₃ | H | H | H | - | ok | 286 |
| 59 | 2-Cl, 4-F, 5-F, H | H | H | Cl | H | H | OH | - | ok | 253* |
| 60 | 2-Cl, 4-F, H, H | H | H | H | F | H | H | - | ok | |
| 61 | 2-Cl, 4-F, 5-F, H | H | H | H | F | H | H | - | ok | |
| 62 | 2-Cl, 4-F, H, H | H | H | H | Cl | H | H | - | ok | |
| 63 | 2-Cl, 4-F, 5-F, H | H | H | H | Cl | H | H | - | ok | |
| 64 | 2-Cl, 4-F, 5-F, H | H | H | H | OCH₃ | H | H | - | ok | |
| 65 | 2-Cl, 4-F, 5-F, H | H | H | H | NO₂ | H | H | - | ok | |
| 66 | 2-Cl, 4-F, 5-F, H | H | H | OCH₂CF₃ | H | H | H | - | ok | |
| 67 | 2-Cl, 4-F, 5-F, H | H | H | OCH₃ | H | H | NO₂ | - | ok | |
| 68 | 2-Cl, 4-F, H, H | H | H | Cl | H | H | OCH₃ | - | ok | |
| 69 | 2-Cl, 4-F, H, H | H | H | O(CH₂)₂CH₃ | H | H | H | - | ok | >300 |
| 70 | 2-Cl, 4-F, H, H | H | H | OCH(CH₃)₂ | H | H | H | - | ok | 269,5 |
| 71 | 2-Cl, 4-F, H, H | H | H | O(CH₂)₃CH₃ | H | H | H | - | ok | 285 |
| 72 | 2-Cl, 4-F, H, H | | | 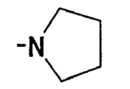 | H | H | H | - | ok | 258 |
| 73 | 2-Cl, 4-F, H, H | H | H | 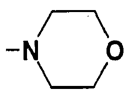 | H | H | H | - | ok | 274 |
| 74 | 2-Cl, 4-Cl, 6-Cl, H | H | H | OCH₃ | H | H | H | - | ok | |
| 75 | 2-Br, 4-CH₃, H, H | H | H | OCH₃ | H | H | H | - | ok | |
| 76 | 2-Br, 4-F, H, H | H | H | OCH₃ | H | H | H | - | ok | |
| 77 | 2-Br, 5-F, H, H | H | H | OCH₃ | H | H | H | - | ok | |
| 78 | 2-F, 4-Cl, 5-F, H | H | H | OCH₃ | H | H | H | - | ok | |
| 79 | 4-Cl, 5-F, H, H | H | H | OCH₃ | H | H | H | - | ok | |
| 80 | 2-Cl, 4-Cl, 6-CH₃, H | H | H | OCH₃ | H | H | H | - | ok | |
| 81 | 2-CF₃, 4-F, H, H | H | H | OCH₃ | H | H | H | - | ok | |
| 82 | 2-CF₃, 6-F, H, H | H | H | OCH₃ | H | H | H | - | ok | |
| 83 | 2-Cl, 3-CH₃, H, H | H | H | OCH₃ | H | H | H | - | ok | |
| 84 | 2-Cl, 6-F, 5-CH₃, H | H | H | OCH₃ | H | H | H | - | ok | |
| 85 | 2-Cl, 6-F, 3-CH₃, H | H | H | OCH₃ | H | H | H | - | ok | |
| 86 | 2-Cl, 4-F, H, H | H | H | N(CH₃)₂ | H | H | H | - | ok | 247 |
| 87 | 2-Cl, 4-F, 5-F, H | H | H | O(CH₂)₂CH₃ | H | H | H | - | ok | 289 |
| 88 | 2-Cl, 4-F, 5-F, H | H | H | O(CH₂)₃CH₃ | H | H | H | - | ok | 275,5 |
| 89 | 2-Cl, 4-F, 5-F, H | H | H | 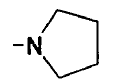 | H | H | H | - | ok | 209,5 |
| 90 | 2-Cl, 4-F, 5-F, H | H | H | 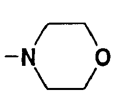 | H | H | H | - | ok | 303 |
| 91 | 2-CH₃, 4-Br, H, H | H | H | OCH₃ | H | H | H | - | ok | |
| 92 | 2-Cl, 4-Br, H, H | H | H | OCH₃ | H | H | H | - | ok | |
| 93 | 2-Br, 5-Cl, H, H | H | H | OCH₃ | H | H | H | - | ok | |
| 94 | 2-CH₃, 5-CH₃, H, H | H | H | OCH₃ | H | H | H | - | ok | |
| 95 | 2-CH₃, 5-F, H, H | H | H | OCH₃ | H | H | H | - | ok | |
| 96 | 2-F, 4-F, 5-F | H | H | OCH₃ | H | H | H | - | ok | |
| 97 | 2-CH₃, 4-F, H, H | H | H | OCH₃ | H | H | H | - | ok | |
| 98 | 2-CH₃, 5-Cl, H, H | H | H | OCH₃ | H | H | H | - | ok | |
| 99 | 2-Cl, 4-F, 5-F, H | H | H | NHCH₂CH₃ | H | H | H | - | ok | 300 |
| 100 | 2-Cl, 4-F, 5-F, H | H | H | OCH₃ | H | H | NH₂ | - | ok | |
| 101 | 2-Cl, 4-F, 5-F, H | H | H | OCH(CH₃)₂ | H | H | H | - | ok | 261 |

| | | | | | | | | | | |
|---|---|---|---|---|---|---|---|---|---|---|
| * Zersetzung ** Unter der Angabe "MS ist ok" wird verstanden, dass ein Massenspektrum oder HPLC/MS gemessen wurde und in diesem der Molpeak (Molmasse + H⁺) nachgewiesen wurde. | | | | | | | | | | |

Als Vergleichsbeispiel A wurde das Beispiel 5 aus EP 0 193 249 synthetisiert. Vergleichsbeispiel A hat die Struktur:

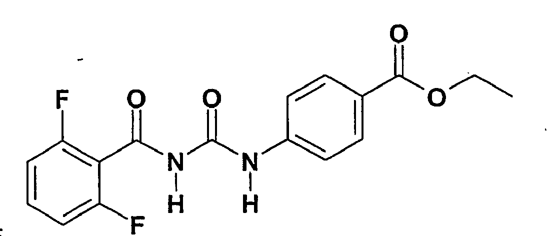

Die Verbindungen der Formel I zeichnen sich durch günstige Wirkungen auf den Zuckerstoffwechsel aus, sie senken insbesondere den Blutzuckerspiegel und sind zur Behandlung von Typ 2 Diabetes geeignet. Die Verbindungen können daher allein oder in Kombination mit weiteren Blutzucker senkenden Wirkstoffen (Antidiabetika) eingesetzt werden.

Die Verbindungen der Formel I eignen sich weiterhin zur Behandlung von Diabetischen Spätschäden, wie z.B. Nephropatie, Retinopathie, Neuropathie sowie Herzinfarkt, Myocardialem Infarkt, peripheren arteriellen Verschlusskrankheiten, Thrombosen, Arteriosklerose, Syndrom X, Obesitas, Entzündungen, Immunkrankheiten, Autoimmunkrankheiten, wie z.B. AIDS, Asthma, Osteoporose, Krebs, Psoriasis, Alzheimer, Schizophrenie und Infektionskrankheiten.

Die Wirksamkeit der Verbindungen wurde wie folgt getestet:

### Glykogenphosphorylase a Aktivitätstest

Der Effekt von Verbindungen auf die Aktivität der aktiven Form der Glykogenphosphorylase (GPa) wurde in der umgekehrten Richtung, durch Verfolgen der Glykogensynthese aus Glukose-1-Phosphat an Hand der Bestimmung der Freisetzung von anorganischem Phosphat, gemessen. Alle Reaktionen wurden als Doppelbestimmungen in Mikrotiterplatten mit 96-Vertiefungen (Half Area Plates, Costar Nr. 3696) durchgeführt, wobei die Änderung der Absorption auf Grund der Bildung des Reaktionsprodukts bei der weiter unten spezifizierten Wellenlänge in einem Multiskan Ascent Elisa Reader (Lab Systems, Finnland) gemessen wurde.

Um die GPa Enzymaktivität in der umgekehrten Richtung zu messen, wurde die Umwandlung von Glukose-1-Phosphat in Glykogen und anorganisches Phosphat nach der allgemeinen Methode von Engers et al. (Engers HD, Shechosky S, Madsen NB, Can J Biochem 1970 Jul;48(7):746-754) mit folgenden Modifikationen gemessen: Humane Glykogenphosphorylase a (zum Beispiel mit 0,76 mg Protein / ml (Aventis Pharma Deutschland GmbH), gelöst in Pufferlösung E (25 mM β-Glyzerophosphat, pH 7,0, 1 mM EDTA und 1 mM Dithiotreitol) wurde mit Puffer T (50 mM Hepes, pH 7,0, 100 mM KCI, 2,5 mM EDTA, 2,5 mM MgCl₂·6H₂O) und Zusatz von 5 mg/ml Glykogen auf eine Konzentration von 10 µg Protein/ml verdünnt. Prüfsubstanzen wurden als 10 mM Lösung in DMSO zubereitet und auf 50 µM mit Pufferlösung T verdünnt. Zu 10 µl dieser Lösung wurden 10 µl 37,5 mM Glukose, gelöst in Pufferlösung T und 5 mg/mL Glykogen, sowie 10 µl einer Lösung von humaner Glykogenphosphorylase a (10 µl Protein/ml) und 20 µl Glukose-1-Phosphat, 2,5 mM zugegeben. Der basale Wert der Glykogenphosphorylase a Aktivität in Abwesenheit von Prüfsubstanz wurde durch Zugabe von 10 µl Pufferlösung T (0,1 % DMSO) bestimmt. Die Mischung wurde 40 Minuten bei Raumtemperatur inkubiert und das freigesetzte anorganische Phosphat mittels der allgemeinen Methode von Drueckes et al. (Drueckes P, Schinzel R, Palm. D, Anal Biochem 1995 Sep 1;230(1):173-177) mit folgenden Modifikationen gemessen: 50 µl einer Stop-Lösung von 7,3 mM Ammoniummolybdat, 10,9 mM Zinkacetat, 3,6 % Askorbinsäure, 0,9 % SDS werden zu 50 µl der Enzymmischung gegeben. Nach 60 Minuten Inkubation bei 45 °C wurde die Absorption bei 820 nm gemessen. Zur Bestimmung der Hintergrundsabsorption wurde in einem separaten Ansatz die Stop-Lösung unmittelbar nach Zugabe der Glukose-1-Phosphatlösung zugegeben.

Dieser Test wurde mit einer Konzentrationen von 10 µM der Prüfsubstanz durchgeführt, um die jeweilige Hemmung der Glykogenphosphorylase a in vitro durch die Prüfsubstanz zu bestimmen.

**Tabelle 2: Biologische Aktivität**

| Bsp. | % Inhibition bei 10 µM | | Bsp. | % Inhibition bei 10 µM |
|---|---|---|---|---|
| 1 | 71 | | 52 | 96 |
| 2 | 85 | | 53 | 93 |
| 3 | 93 | | 54 | 91 |
| 4 | 56 | | 55 | 100 |
| 5 | 80 | | 56 | 96 |
| 6 | 89 | | 57 | 99 |
| 7 | 93 | | 58 | 91 |
| 8 | 96 | | 59 | 92 |
| 9 | 100 | | 60 | 41 |
| 10 | 95 | | 61 | 85 |
| 11 | 95 | | 62 | 59 |
| 12 | 82 | | 63 | 92 |
| 13 | 74 | | 64 | 40 |
| 14 | 70 | | 65 | 56 |
| 15 | 90 | | 66 | 97 |
| 16 | 89 | | 67 | 92 |
| 17 | 75 | | 68 | 54 |
| 18 | 64 | | 69 | 99 |
| 19 | 94 | | 70 | 100 |
| 20 | 85 | | 71 | 95 |
| 21 | 81 | | 72 | 99 |
| 22 | 79 | | 73 | 85 |
| 23 | 59 | | 74 | 47 |
| 24 | 87 | | 75 | 84 |
| 25 | 82 | | 76 | 98 |
| 26 | 81 | | 77 | 96 |
| 27 | 90 | | 78 | 69 |
| 28 | 91 | | 79 | 58 |
| 29 | 72 | | 80 | 65 |
| 30 | 95 | | 81 | 49 |
| 31 | 98 | | 82 | 40 |
| 32 | 98 | | 83 | 34 |
| 33 | 100 | | 84 | 98 |
| 34 | 59 | | 85 | 98 |
| 35 | 94 | | 86 | 99 |
| 36 | 96 | | 87 | 102 |
| 37 | 91 | | 88 | 99 |
| 38 | 103 | | 89 | 102 |
| 39 | 98 | | 90 | 95 |
| 40 | 92 | | 91 | 94 |
| 41 | 101 | | 92 | 95 |
| 42 | 99 | | 93 | 96 |
| 43 | 100 | | 94 | 88 |
| 44 | 101 | | 95 | 96 |
| 45 | 96 | | 96 | 90 |
| 46 | 92 | | 97 | 97 |
| 47 | 98 | | 98 | 95 |
| 48 | 99 | | 99 | 95 |
| 49 | 103 | | 100 | 95 |
| 50 | 108 | | 101 | 100 |
| 51 | 96 | | | |

### Vergleichsbeispiel A zeigt 3% Inhibition bei 10 µM.

Aus der Tabelle ist abzulesen, dass die Verbindungen der Formel I die Aktivität der Glykogenphosphorylase a hemmen und dadurch zur Senkung des Blutzuckerspiegels gut geeignet sind. Insbesondere weisen die Verbindungen der Formel I eine 14-bis 36-fach erhöhte Wirkung gegenüber dem Vergleichsbeispiel A auf.

Nachfolgend wird die Herstellung eines Beispiels detailliert beschrieben, die übrigen Verbindungen der Formel I wurden analog, ggf. unter Verwendung üblicher Schutzgruppentechniken, erhalten:

### Experimenteller Teil:

### Beispiel 1:

### a) 2-Chlorbenzoylisocyanat

1,03 g (6,6 mmol) 2-Chlorbenzamid wurde in 3 ml Dichlormethan suspendiert. Nach Zugabe von 1,17 g (9,2 mmol) Oxalylchlorid wurde 17 Stunden zum Rückfluss erhitzt. Das Reaktionsgemisch wurde am Hochvakuum eingeengt und ohne weitere Reinigung in Stufe b umgesetzt.

### b) 3-[3-(2,4-Dichloro-benzoyl)-ureido]-4-methoxy-benzoesäure

2-Chlorbenzoylisocyanat (Stufe a) wurde in 8 ml Acetonitril aufgenommen und mit einer Suspension von 1,1 g (6 mmol) 4-Amino-3-nitro-benzoesäure in 24 ml Acetonitril versetzt. Es wurde 3,5 Stunden zum Rückfluss erhitzt, nach dem Abkühlen wurde der Niederschlag abfiltriert, mit Acetonitril gewaschen und im Vakuum getrocknet. Man erhielt 1,68 g (77%) des gewünschten Produktes.
Smp.: 240°C (Zersetzung)

### Beispiel 2:

### a) 4-[3-(2-Chlor-4,5-difluor-benzoyl)-ureido]-3-trifluoromethoxy-benzoesäure

### Die Herstellung der Verbindung erfolgte in einer Eintopfreaktion.

30,0 g (155,8 mmol) 2-Chlor-4,5-difluorbenzoesäure wurden in einem 1l Rundkolben mit gasdichtem mechanischen Rührer und Destillationsaufsatz unter Schutzgasatmosphäre vorgelegt. Nach Zugabe von 300 ml Toluol wurden 29,01 ml Thionylchlorid unter Rühren zugegeben und der Ansatz auf 60°C erhitzt. Bei 60°C wurde die Reaktionsmischung 1,5 h nachgerührt und dann mit 0,1 ml Pyridin versetzt. Nach weiteren 1,5 h bei 60°C wurden aus dem Ansatz bei Normaldruck 90 ml Flüssigkeit abdestilliert (maximale Badtemperatur 125°C). Die Reaktionslösung wurde anschließend auf 20°C abgekühlt und bei 20-35°C (Kühlung mit Eisbad) Ammoniak-Gas bis zur Sättigung der Lösung eingeleitet. Daraufhin wurden bei 20°C 160 ml THF sowie 120 ml entionisiertes Wasser zum Ansatz gegeben. Die wässrige Phase wurde abgetrennt und die organische Phase mit 5%iger, wässriger

Natriumhydrogencarbonatlösung gewaschen. Die organische Phase wurde dann durch abdestillieren von 250 ml Flüssigkeit im Vakuum bei 50°C azeotrop getrocknet. Zu der auf 20°C abgekühlten Reaktionssuspension wurden 17 ml Oxalylchlorid gegeben. Der Ansatz wurde bei 20°C 2h nachgerührt und anschließend 4h bei 50°C gerührt. Aus dem Ansatz wurden im Vakuum bei 50°C 200 ml Flüssigkeit abdestilliert dann mit 200 ml Toluol versetzt und nochmals im Vakuum bei 50°C 200 ml Flüssigkeit abdestilliert. Der Ansatz wurde auf 20°C abgekühlt und mit einer Lösung von 26,49 g (119,8 mmol) 4-Amino-3-(trifluormethoxy)benzoesäure in 75ml THF versetzt. Das Produkt wurde durch Filtration über eine Glasfilternutsche abgetrennt und im Vakuum bei 50°C bis zur Gewichtskonstanz getrocknet. Es wurden 52,6g des gewünschten Produktes erhalten.
Smp.: 236°C

### b) 4-[3-(2-Chlor-4,5-difluor-benzoyl)-ureido]-3-trifluoromethoxy-benzoesäure TRIS-Salz

Eine Mischung aus 10,0g (22,79 mmol) 4-[3-(2-Chlor-4,5-difluor-benzoyl)-ureido]-3-trifluoromethoxy-benzoesäure und 3,0 g α,α,α- Tris-(hydroxymethyl)-methylamin wurden in 400 ml 2-Propanol zum Rückfluß erhitzt, bis eine klare Lösung entstand. Die Lösung wurde heiß filtriert und auf 310 ml Volumen eingeengt. Beim Abkühlen auf 20°C kristallisierte das Produkt aus der Lösung aus. Es wurden 10,4g des gewünschten Produktes erhalten.
Smp.: 176°C

Die Verbindungen der Formel I können hergestellt werden, dadurch dass Harnstoffe der Formel 2 oder Anilinderivate der Formel 3 mit Aroyl-isocyanaten, mit reaktiven Säurederivaten, mit Säurechloriden oder mit Anhydriden, der Formel 4, 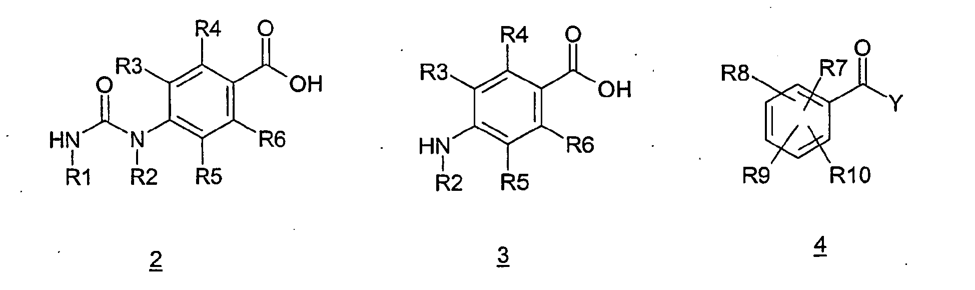 worin R1 bis R6 die oben angegebenen Bedeutungen haben, umgesetzt werden. Die resultierenden freien Säuren der Formel I können dann mit den entsprechenden Basen zu den entsprechenden physiologisch verträglichen Salzen der Verbindungen der Formel I ungesetzt werden.
Zur Erläuterung: Falls Formel 4 ein Säurechlorid darstellt, ist Y gleiche Cl, falls 4 ein Isocyanat darstellt ist Y gleich N=C=O und falls 4 ein Anhydrid darstellt ist Y gleich

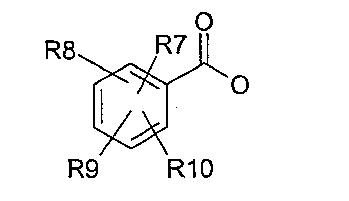

Bevorzugt ist der das Herstellungsverfahren der Verbindungen der Formel I über das Aroyl-isocyanat 4a, wie in folgendem Schema dargestellt:

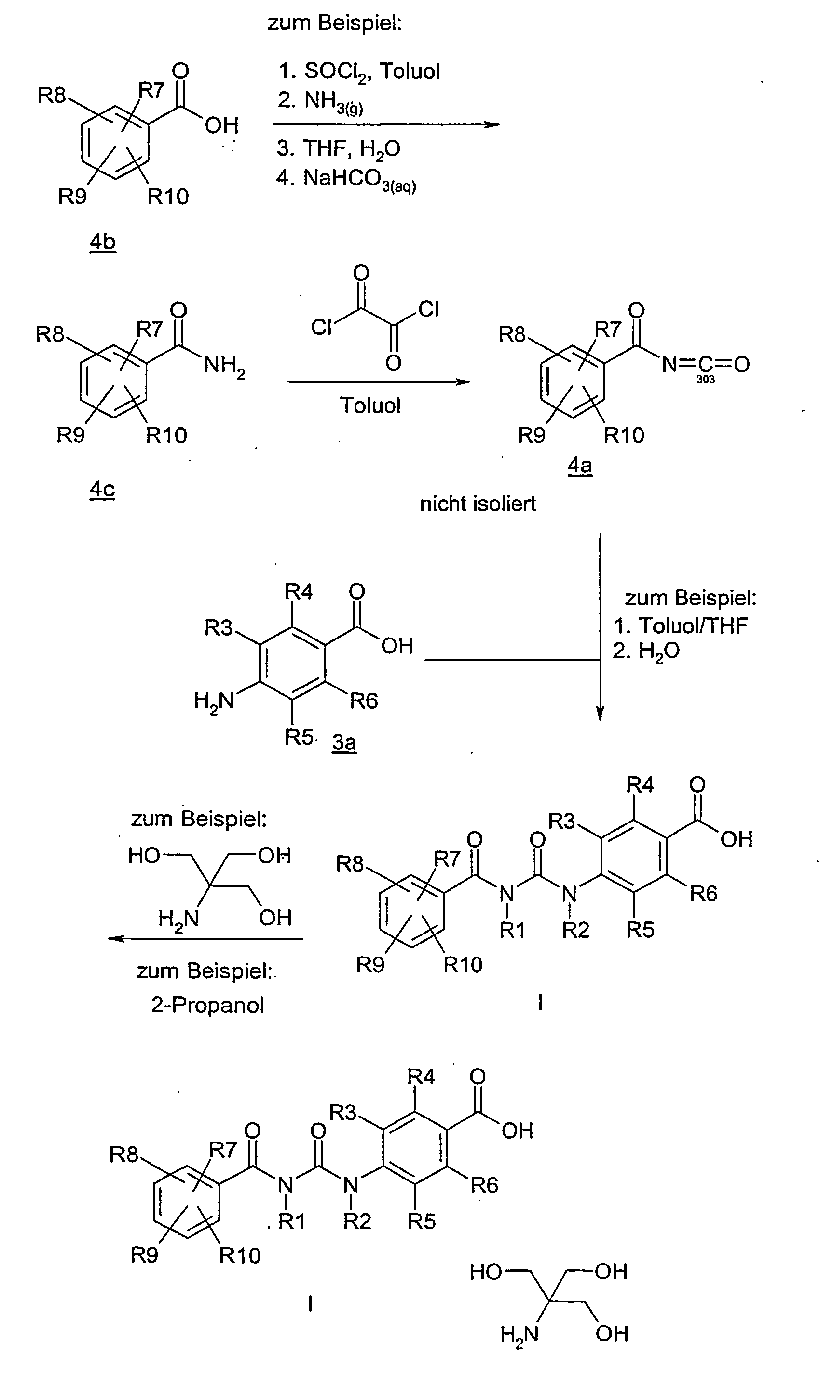

Die Herstellung von I kann generell in einem Eintopfverfahren geschehen, was die großtechnische Herstellung stark vereinfacht. 4b wird dazu in einem geeigneten Lösemittel, wie z.B. Toluol, mittels eines geeigneten Reagenzes, wie z.B. Thionylchlorid oder Oxalylchlorid, in das Säurechlorid überführt. Hierbei wird je nach Bedarf zur Vervollständigung der Reaktion ein geeigneter Katalysator, wie z.B. Pyridin, NMP oder DMF, zugesetzt. Nach Entfernen des nicht abreagierten Reagenzes (wie z.B. Thionylchlorid) wird das Säurechlorid durch geeignete Reagenzien, wie z.B. das Einleiten von Ammoniak-Gas in die Reaktionslösung, oder die Zugabe einer Lösung von Ammoniak in einem geeigneten Lösemittel, wie z.B. THF, in das Säureamid 4b überführt. Die Reaktionsmischung wird mit soviel Wasser und einem geeigneten Lösemittel, wie z.B. THF, so versetzt, daß jeglicher Feststoff in Lösung geht. Nach der Phasentrennung wird mit Natriumhydrbgencarbonatlösung gewaschen und die organische Phase anschließend getrocknet. Durch Zugabe von Oxalylchlorid und anschließendes Erhitzen wird das Säureamid 4b in das Aroyl-isocyanat 4a überführt. Nach Entfernen des nicht abreagierten Oxalylchlorids wird das Anilin 3a in einem geeigneten Lösemittel, wie z.B. THF, gelöst und zur Lösung des Aroyl-isocyanates 4a zugegeben, wobei das Reaktionsprodukt aus der Lösung ausfällt. Die Verbindung der Formel I wird durch Filtration abgetrennt. Das TRIS-Salz der Verbindung der Formel I kristallisiert nach. Lösen der Verbindung der Formel I mit TRIS in einem geeigneten Lösemittel, wie z.B. 2-Propanol, in der Siedehitze beim anschließenden Abkühlen der Lösung aus.

## Patentansprüche

1. Verbindungen der Formel I, 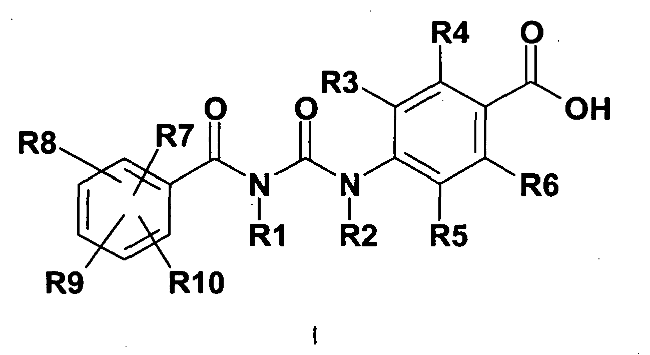 worin bedeuten
R7, R8, R9, R10 unabhängig von einander H, F, Cl, Br, OH, NO₂, CN, O-(C₁- C₆)-Alkyl, O-(C₂-C₆)-Alkenyl, O-(C₂-C₆)-Alkinyl, O-SO₂-(C₁-C₄)-Alkyl, (C₁- C₆)-Alkyl, (C₂-C₆)-Alkenyl, (C₂-C₆)-Alkinyl, wobei Alkyl, Alkenyl und Alkinyl ein oder mehrfach durch F, Cl oder Br substituiert sein können;
R1, R2 unabhängig voneinander H, (C₁-C₆)-Alkyl, wobei Alkyl mit OH, O-(C₁- C₄)-Alkyl, NH₂, NH(C₁-C₄)-Alkyl, N[(C₁-C₆)-Alkyl]₂ substituiert sein kann, O-(C₁-C₆)-Alkyl, CO-(C₁-C₆)-Alkyl, COO-(C₁-C₆)-Alkyl, (C₁-C₆)-Alkylen- COOH oder (C₁-C₆)-Alkylen-COO-(C₁-C₆)-alkyl;
R3 H, F, Cl, Br, NO₂, CN, O-R11, O-Phenyl, S-R11, COOR11, N(R12)(R13), (C₁-C₆)-Alkyl, (C₂-C₆)-Alkenyl, (C₂-C₆)-Alkinyl, (C₃-C₇)- Cycloalkyl oder (C₃-C₇)-Cycloalkyl-(C₁-C₄)-alkylen, wobei Alkyl, Cycloalkyl und Alkinyl ein oder mehrfach mit F, Cl, Br, OR11, COOR11 oder N(R16)(R17) substituiert sein können;
R4 H, F, Cl, Br, NO₂, CN, O-R11, O-Phenyl, S-R11, COOR11, N(R12)(R13), (C₁-C₆)-Alkyl, (C₂-C₆)-Alkenyl, (C₂-C₆)-Alkinyl, (C₃-C₇)- Cycloalkyl oder (C₃-C₇)-Cycloalkyl-(C₁-C₄)-alkylen, wobei Alkyl, Cycloalkyl und Alkinyl ein oder mehrfach mit F, Cl, Br, OR11, COOR11 oder N(R16)(R17) substituiert sein können;
R5 H, F, Cl, Br, NO₂, CN, O-R11, O-Phenyl, S-R11, COOR11, N(R12)(R13), (C₁-C₆)-Alkyl, (C₂-C₆)-Alkenyl, (C₂-C₆)-Alkinyl, (C₃-C₇)- Cycloalkyl oder (C₃-C₇)-Cycloalkyl-(C₁-C₄)-alkylen, wobei Alkyl, Cycloalkyl und Alkinyl ein oder mehrfach mit F, Cl, Br, OR11, COOR11 oder N(R16)(R17) substituiert sein können;
R6 H, F, Cl, Br, NO₂, CN, O-R11, O-Phenyl, S-R11, COOR11, N(R12)(R13), (C₁-C₆)-Alkyl, (C₂-C₆)-Alkenyl, (C₂-C₆)-Alkinyl, (C₃-C₇)- Cycloalkyl oder (C₃-C₇)-Cycloalkyl-(C₁-C₄)-alkylen, wobei Alkyl, Cycloalkyl und Alkinyl ein oder mehrfach mit F, Cl, Br, OR11, COOR11 oder N(R16)(R17) substituiert sein können;
R11 H, (C₁-C₈)-Alkyl, (C₂-C₈)-Alkenyl oder (C₂-C₈)-Alkinyl, wobei Alkyl, Alkenyl und Alkinyl ein oder mehrfach mit F, Cl, Br, OH oder O-(C₁-C₄)- Alkyl substituiert sein können;
R12, R13 unabhängig voneinander H, (C₁-C₈)-Alkyl, (C₂-C₈)-Alkenyl, (C₂-C₈)- Alkinyl, (C₃-C₇)-Cycloalkyl, (C₃-C₇)-Cycloalkyl-(C₁-C₄)-alkylen, COO-(C₁- C₄)-Alkyl, COO-(C₂-C₄)-Alkenyl, Phenyl oder SO₂-Phenyl, wobei der Phenylring bis zu zweifach mit F, Cl, CN, OH, (C₁-C₆)-Alkyl, O-(C₁-C₆)- Alkyl, CF₃, OCF₃, COOH, COO-(C₁-C₆)-Alkyl oder CONH₂ substituiert sein kann;
oder R12 und R13 bilden gemeinsam mit dem Stickstoffatom an das sie gebunden sind einen 3-7 gliedrigen, gesättigten heterozyklischen Ring, der bis zu 2 weitere Heteroatome aus der Gruppe N, O oder S enthalten kann und wobei der heterozyklische Ring bis zu vierfach mit F, Cl, Br, OH, Oxo, (C₁-C₄)-Alkyl oder N(R14)(R15) substituiert sein kann;
R14, R15 unabhängig voneinander H, (C₁-C₈)-Alkyl, (C₂-C₈)-Alkenyl, (C₂-C₈)- Alkinyl, (C₃-C₇)-Cycloalkyl, (C₃-C₇)-Cycloalkyl-(C₁-C₄)-alkylen, COO-(C₁- C₄)-Alkyl, COO-(C₂-C₄)-Alkenyl, Phenyl oder SO₂-Phenyl, wobei der Phenylring bis zu zweifach mit F, Cl, CN, OH, (C₁-C₆)-Alkyl, O-(C₁-C₆)- Alkyl, CF₃, OCF₃, COOH, COO(C₁-C₆)-Alkyl oder CONH₂ substituiert sein kann;
R16, R17 unabhängig voneinander H, (C₁-C₈)-Alkyl, (C₂-C₈)-Alkenyl, (C₂-C₈)- Alkinyl, (C₃-C₇)-Cycloalkyl, (C₃-C₇)-Cycloalkyl-(C₁-C₄)-alkylen, COO-(C₁- C₄)-Alkyl, COO-(C₂-C₄)-Alkenyl, Phenyl oder SO₂-Phenyl, wobei der Phenylring bis zu zweifach mit F, Cl, CN, OH, (C₁-C₆)-Alkyl, O-(C₁-C₆)- Alkyl, CF₃, OCF₃, COOH, COO-(C₁-C₆)-Alkyl oder CONH₂ substituiert sein kann;
oder R16 und R17 bilden gemeinsam mit dem Stickstoffatom an das sie gebunden sind einen 3-7 gliedrigen, gesättigten heterozyklischen Ring, der bis zu 2 weitere Heteroatome aus der Gruppe N, O oder S enthalten kann und wobei der heterozyklische Ring bis zu vierfach mit F, Cl, Br, OH, Oxo, (C₁-C₄)-Alkyl oder N(R14)(R15) substituiert sein kann;
wobei immer mindestens einer der Reste R3, R7, R8, R9 und R10 ungleich
Wasserstoff ist und
wobei die Verbindung 4-[3-(2,4-Dichloro-benzoyl)-ureido]-3-methoxy-benzoesäure
ausgeschlossen ist;
sowie deren physiologisch verträgliche Salze.

2. Verbindungen der Formel I, gemäß Anspruch 1, **dadurch gekennzeichnet, dass** darin bedeuten
R7, R8, R9, R10 unabhängig von einander H, F, Cl, Br, OH, NO₂, CN, (C₁-C₆)- Alkyl oder O-(C₁-C₆)-Alkyl, wobei Alkyl ein oder mehrfach durch F substituiert sein kann;
R1, R2 H;
R3 H, F, Cl, Br, NO₂, CN, O-R11, O-Phenyl, S-R11, COOR11, N(R12)(R13), (C₁-C₆)-Alkyl, (C₂-C₆)-Alkenyl, (C₂-C₆)-Alkinyl, (C₃-C₇)- Cycloalkyl oder (C₃-C₇)-Cycloalkyl-(C₁-C₄)-alkylen, wobei Alkyl, Cycloalkyl und Alkinyl ein oder mehrfach mit F, Cl, Br, OR11 oder COOR11 substituiert sein können;
R4 H, F, Cl, NO₂, O-R11, N(R12)(R13) oder (C₁-C₆)-Alkyl, wobei Alkyl ein oder mehrfach mit F substituiert sein kann;
R5 H, F, Cl, NO₂, O-R11, N(R12)(R13) oder (C₁-C₆)-Alkyl, wobei Alkyl ein oder mehrfach mit F substituiert sein kann;
R6 H, F, Cl, NO₂, O-R11, N(R12)(R13) oder (C₁-C₆)-Alkyl, wobei Alkyl ein oder mehrfach mit F substituiert sein kann;
R11 H, (C₁-C₈)-Alkyl, (C₁-C₈)-Alkylen-O-(C₁-C₈)-Alkyl oder (C₁-C₈)-Alkyl-OH, wobei Alkyl ein oder mehrfach durch F substituiert sein kann;
R12, R13 unabhängig voneinander H oder (C₁-C₈)-Alkyl;
oder R12 und R13 bilden gemeinsam mit dem Stickstoffatom an das sie gebunden sind einen 3-7 gliedrigen, gesättigten heterozyklischen Ring, der bis zu 2 weitere Heteroatome aus der Gruppe N, O oder S enthalten kann und wobei der heterozyklische Ring bis zu vierfach mit F, Cl, Br, OH, Oxo, (C₁-C₄)-Alkyl oder N(R14)(R15) substituiert sein kann;
R14, R15 unabhängig voneinander H, (C₁-C₈)-Alkyl, (C₂-C₈)-Alkenyl, (C₂-C₈)- Alkinyl, (C₃-C₇)-Cycloalkyl, (C₃-C₇)-Cycloalkyl-(C₁-C₄)-alkylen, COO-(C₁- C₄)-Alkyl, COO-(C₂-C₄)-Alkenyl, Phenyl oder SO₂-Phenyl, wobei der Phenylring bis zu zweifach mit F, Cl, CN, OH, (C₁-C₆)-Alkyl, O-(C₁-C₆)- Alkyl, CF₃, OCF₃, COOH, COO(C₁-C₆)-Alkyl oder CONH₂ substituiert sein kann;
wobei die Verbindung 4-[3-(2,4-Dichloro-benzoyl)-ureido]-3-methoxy-benzoesäure
ausgeschlossen ist;
sowie deren physiologisch verträgliche Salze.

3. Verbindungen der Formel I, gemäß Anspruch 1 oder 2, **dadurch gekennzeichnet, dass** darin bedeuten
R7, R8, R9, R10 unabhängig von einander H, F, Cl, Br, CH₃ oder CF₃;
R1, R2, R5 H;
R3 H, F, Cl, NO₂, CF₃, O-R11, N(R12)(R13) oder (C₁-C₆)-Alkyl, wobei Alkyl ein oder mehrfach mit F substituiert sein kann;
R4 H, F, Cl, NO₂, CF₃, O-R11, N(R12)(R13) oder (C₁-C₆)-Alkyl, wobei Alkyl ein oder mehrfach mit F substituiert sein kann;
R6 H, F, Cl, NO₂, CF₃, O-R11, N(R12)(R13) oder (C₁-C₆)-Alkyl, wobei Alkyl ein oder mehrfach mit F substituiert sein kann;
R11 H oder (C₁-C₈)-Alkyl, wobei Alkyl ein oder mehrfach mit F substituiert sein kann,
R12, R13 H oder (C₁-C₈)-Alkyl;
oder die beiden Reste R12 und R13 gemeinsam mit dem Stickstoffatom an das sie
gebunden sind einen 5-6 gliedrigen, gesättigten heterozyklischen Ring
bilden, der ein weiteres Sauerstoffatom enthalten kann;
wobei die Verbindung 4-[3-(2,4-Dichloro-benzoyl)-ureido]-3-methoxy-benzoesäure
ausgeschlossen ist;
sowie deren physiologisch verträgliche Salze.

4. Verbindungen der Formel I, gemäß einem oder mehreren der Ansprüche 1 bis 3, **dadurch gekennzeichnet, dass** darin bedeuten
R7, R8, R9, R10 unabhängig von einander H, F, Cl, Br, CH₃ oder CF₃;
R1, R2, R4, R5, R6 H;
R3 H, F, Cl, NO₂, O-R11, N(R12)(R13) oder (C₁-C₆)-Alkyl, wobei Alkyl ein oder mehrfach mit F substituiert sein kann;
R11 H oder (C₁-C₈)-Alkyl, wobei Alkyl ein oder mehrfach mit F substituiert sein kann,
R12, R13 H oder (C₁-C₈)-Alkyl;
oder die beiden Reste R12 und R13 bilden mit dem Stickstoffatom an das sie
gebunden sind einen 5-6 gliedrigen, gesättigten heterozyklischen Ring, der ein weiteres Sauerstoffatom enthalten kann;
wobei die Verbindung 4-[3-(2,4-Dichloro-benzoyl)-ureido]-3-methoxy-benzoesäure ausgeschlossen ist;
sowie deren physiologisch verträgliche Salze

5. Verbindungen der Formel I, gemäß einem oder mehreren der Ansprüche 1 bis 4, **dadurch kennzeichnet, dass** es sich jeweils um das Trometamolsalz handelt.

6. Arzneimittel enthaltend eine oder mehrere der Verbindungen gemäß einem oder mehreren der Ansprüche 1 bis 5.

7. Arzneimittel enthaltend eine oder mehrere der Verbindungen gemäß einem oder mehreren der Ansprüche 1 bis 5 und ein oder mehrere Blutzucker senkende Wirkstoffe.

8. Arzneimittel enthaltend eine oder mehrere der Verbindungen gemäß einem oder mehreren der Ansprüche 1 bis 5 und ein oder mehrere Statine.

9. Verwendung der Verbindungen gemäß einem oder mehreren der Ansprüche 1 bis 5 zur Herstellung eines Medikamentes zur Behandlung des Typ 2 Diabetes.

10. Verwendung der Verbindungen gemäß einem oder mehreren der Ansprüche 1 bis 5 zur Herstellung eines Medikamentes zur Blutzuckersenkung.

11. Verwendung der Verbindungen gemäß einem oder mehreren der Ansprüche 1 bis 5 in Kombination mit mindestens einem weiteren Blutzucker senkenden Wirkstoff zur Herstellung eines Medikamentes zur Behandlung des Typ 2 Diabetes.

12. Verwendung der Verbindungen gemäß einem oder mehreren der Ansprüche 1 bis 5 in Kombination mit mindestens einem weiteren Blutzucker senkenden Wirkstoff zur Herstellung eines Medikamentes zur Blutzuckersenkung.

13. Verfahren zur Herstellung eines Arzneimittels enthaltend eine oder mehrere der Verbindungen gemäß einem oder mehreren der Ansprüche 1 bis 5, **dadurch gekennzeichnet, dass** der Wirkstoff mit einem pharmazeutisch geeigneten Träger vermischt wird und diese Mischung in eine für die Verabreichung geeignete Form gebracht wird.

14. Verfahren zur Herstellung der Verbindungen der Formel I, **dadurch gekennzeichnet dass** Harnstoffe der Formel 2 oder Anilinderivate der Formel 3 mit Aroyl-isocyanaten, mit reaktiven Säurederivaten, mit Säurechloriden oder mit Anhydriden, der Formel 4, 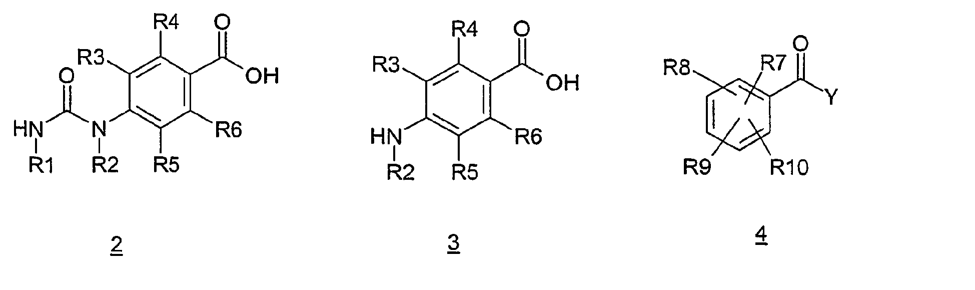 worin R1 bis R10 die in Anspruch 1 angegebenen Bedeutungen haben umgesetzt werden.

15. Verfahren zur Herstellung der Verbindungen der Formel I gemäß Anspruch 14, **dadurch gekennzeichnet dass** Aroyl-isocyanat der Formel 4a mit Säure der Formel 3a, 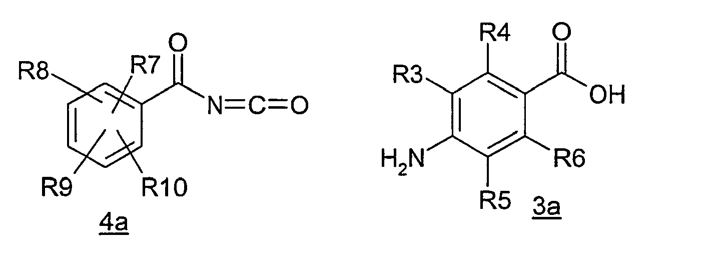 worin R1 bis R10 die in Anspruch 1 angegebenen Bedeutungen haben umgesetzt werden.

16. Verfahren zur Herstellung der physiologisch verträglichen Salze der Verbindungen der Formel I, **dadurch gekennzeichnet dass** die freien Säuren der Verbindungen der Formel I mit einer entsprechenden Base umgesetzt werden.

17. Verfahren nach Anspruch 16, **dadurch gekennzeichnet, dass** die Base α,α,α-Tris-(hydroxymethyl)-methylamin (Tromethamol) ist.

18. Verfahren zur Herstellung der Verbindungen der Formel I, **dadurch gekennzeichnet dass** nach folgendem Reaktionsschema, worin R1 bis R10 die in Anspruch 1 angegebenen Bedeutungen haben, 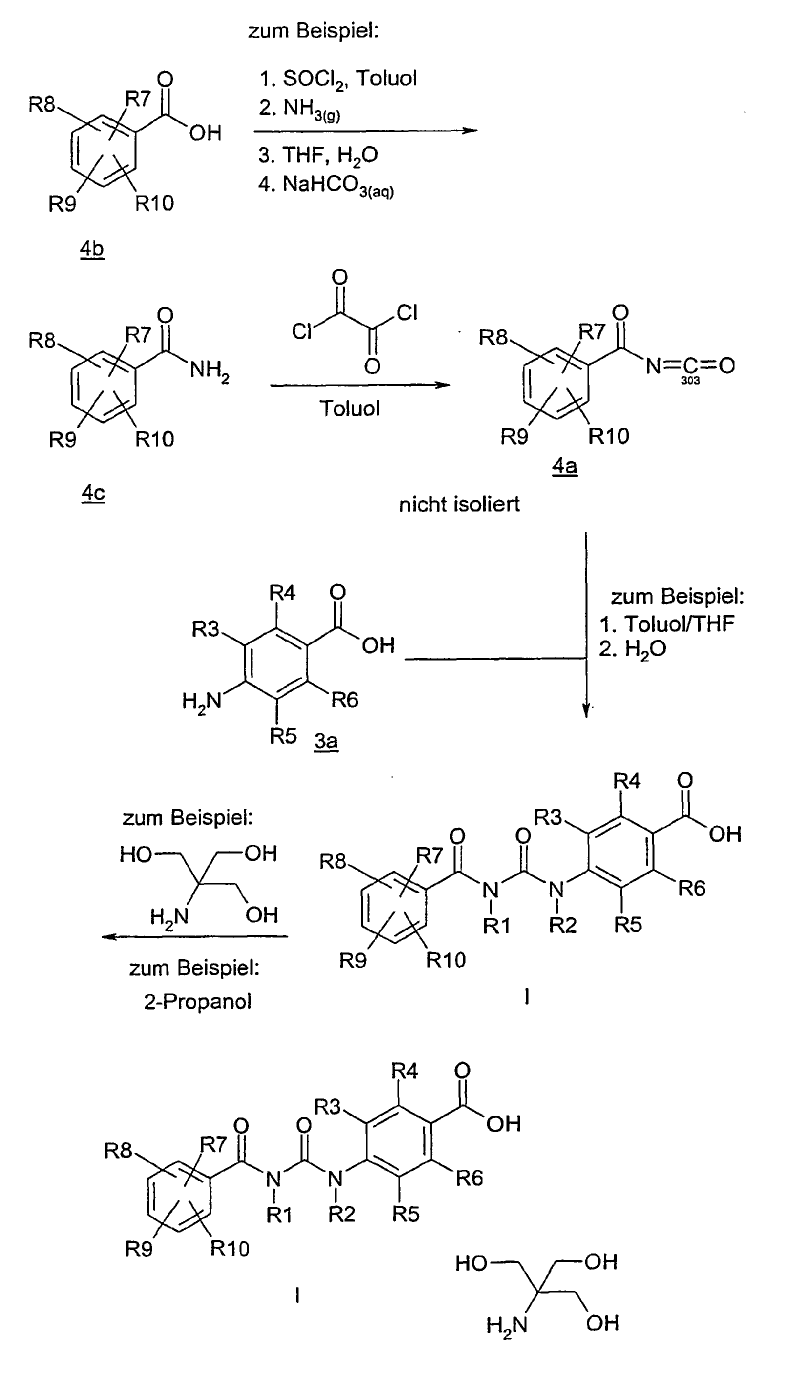 in einem ersten Schritt die Verbindung 4b, in einem dazu geeigneten Lösemittel, mittels eines geeigneten Reagenzes, in das Säurechlorid überführt wird und
in einem zweiten Schritt das so erhaltene Säurechlorid in einem dazu geeigneten Lösemittel, mittels eines geeigneten Reagenzes in das Säureamid 4b überführt wird und
in einem dritten Schritt das Säureamid 4b durch Umsetzen mit Oxalylchlorid in das Aroyl-isocyanat 4a überführt wird und
in einem vierten Schritt das Aroyl-isocyanat 4a mit dem Anilin 3a in einem geeigneten Lösemittel zur freien Säure der Verbindung der Formel I umsetzt wird und
in einem fünften Schritt die freie Säure der Verbindung der Formel I mit einer Base zu einem physiologisch verträglichen Salz der Verbindung der Formel I umgesetzt wird.

## Claims

1. A compound of the formula I, 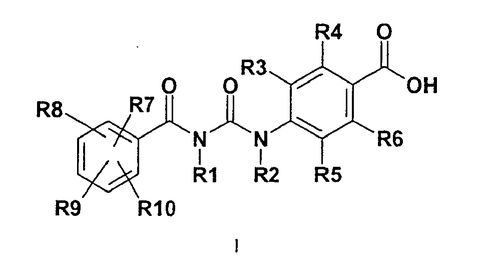 in which
R7, R8, R9 and R10 are, independently of each other, H, F, Cl, Br, OH, NO₂, CN, O-(C₁-C₆)-alkyl, O-(C₂- C₆)-alkenyl, O-(C₂-C₆)-alkynyl, O-SO₂-(C₁-C₄)- alkyl, (C₁-C₆)-alkyl, (C₂-C₆)-alkenyl or (C₂- C₆)-alkynyl, where alkyl, alkenyl and alkynyl can be substituted, once or more than once, by F, Cl or Br;
R1 and R2 are, independently of each other, H, (C₁-C₆)- alkyl, where alkyl can be substituted by OH, O-(C₁-C₄)-alkyl, NH₂, NH (C₁-C₄)-alkyl or N[(C₁- C₆)-alkyl]₂, O-(C₁-C₆)-alkyl, CO-(C₁-C₆)-alkyl, COO-(C₁-C₆)-alkyl, (C₁-C₆)-alkylene-COOH or (C₁-C₆)-alkylene-COO-(C₁-C₆)-alkyl;
R3 is H, F, Cl, Br, NO₂, CN, O-R11, O-phenyl, S-R11, COOR11, N(R12)(R13), (C₁-C₆)-alkyl, (C₂-C₆)- alkenyl, (C₂-C₆)-alkynyl, (C₃-C₇)-cycloalkyl or (C₃-C₇)-cycloalkyl-(C₁-C₄)-alkylene, where alkyl, cycloalkyl and alkynyl can be substituted, once or more than once, by F, Cl, Br, OR11, COOR11 or N(R16)(R17);
R4 is H, F, Cl, Br, NO₂, CN, O-R11, O-phenyl, S-R11, COOR11, N(R12)(R13), (C₁-C₆)-alkyl, (C₂-C₆)- alkenyl, (C₂-C₆)-alkynyl, (C₃-C₇)-cycloalkyl or (C₃-C₇)-cycloalkyl-(C₁-C₄)-alkylene, where alkyl, cycloalkyl and alkynyl can be substituted, once or more than once, by F, Cl, Br, OR11, COOR11 or N(R16)(R17);
R5 is H, F, Cl, Br, NO₂, CN, O-R11, O-phenyl, S-R11, COOR11, N(R12)(R13), (C₁-C₆)-alkyl, (C₂-C₆)- alkenyl, (C₂-C₆)-alkynyl, (C₃-C₇) -cycloalkyl or (C₃-C₇)-cycloalkyl-(C₁-C₄)-alkylene, where alkyl, cycloalkyl and alkynyl can be substituted, once or more than once, by F, Cl, Br, OR11, COOR11 or N(R16)(R17);
R6 is H, F, Cl, Br, NO₂, CN, O-R11, O-phenyl, S-R11, COOR11, N(R12)(R13), (C₁-C₆)-alkyl, (C₂-C₆)- alkenyl, (C₂-C₆)-alkynyl, (C₃-C₇) -cycloalkyl or (C₃-C₇)-cycloalkyl-(C₁-C₄)-alkylene, where alkyl, cycloalkyl and alkynyl can be substituted, once or more than once, by F, Cl, Br, OR11, COOR11 or N(R16)(R17);
R11 is H, (C₁-C₈)-alkyl, (C₂-C₈)-alkenyl or (C₂-C₈)- alkynyl, where alkyl, alkenyl and alkynyl can be substituted, once or more than once, by F, Cl, Br, OH or O-(C₁-C₄)-alkyl;
R12 and R13 are, independently of each other, H, (C₁-C₈)-alkyl, (C₂-C₈)-alkenyl, (C₂-C₈)- alkynyl, (C₃-C₇)-cycloalkyl, (C₃-C₇)- cycloalkyl-(C₁-C₈)-alkylene, COO-(C₁-C₄)-alkyl, COO-(C₂-C₄)-alkenyl, phenyl or SO₂-phenyl, where the phenyl ring can be substituted, up to two times, by F, Cl, CN, OH, (C₁-C₆)-alkyl, O-(C₁-C₆)-alkyl, CF₃, OCF₃, COOH, COO-(C₁-C₆)- alkyl or CONH₂;
or R12 and R13 form, together with the nitrogen atom to which they are bonded, a 3-7-membered, saturated heterocyclic ring which can contain up to 2 further heteroatoms from the group N, O or S and where the heterocyclic ring can be substituted, up to four times, by F, Cl, Br, OH, Oxo, (C₁-C₄) -alkyl or N(R14)(R15);
R14 and R15 are, independently of each other, H, (C₁-C₈)-alkyl, (C₂-C₈)-alkenyl, (C₂-C₈)- alkynyl, (C₃-C₇)-cycloalkyl, (C₃-C₇)- cycloalkyl-(C₁-C₄)-alkylene, COO-(C₁-C₄)-alkyl, COO-(C₂-C₄)-alkenyl, phenyl or SO₂-phenyl, where the phenyl ring can be substituted, up to two times, by F, Cl, CN, OH, (C₁-C₆)-alkyl, O-(C₁-C₆)-alkyl, CF₃, OCF₃, COOH, COO(C₁-C₆)- alkyl or CONH₂;
R16 and R17 are, independently of each other, H, (C₁-C₈)-alkyl, (C₂-C₈)-alkenyl, (C₂-C₈)- alkynyl, (C₃-C₇)-cycloalkyl, (C₃-C₇)- cycloalkyl- (C₁-C₄)-alkylene, COO- (C₁-C₄) -alkyl, COO- (C₂-C₄)-alkenyl, phenyl or SO₂-phenyl, where the phenyl ring can be substituted, up to two times, by F, Cl, CN; OH, (C₁-C₆)-alkyl, O-(C₁-C₆)-alkyl, CF₃ , OCF₃, COOH, COO-(C₁-C₆)- alkyl or CONH ;
or R16 and R17 form, together with the nitrogen atom to which they are bonded, a 3-7-membered, saturated heterocyclic ring which can contain up to 2 further heteroatoms from the group N, O or S and where the heterocyclic ring can be substituted, up to four times, by F, Cl, Br, OH, Oxo, (C₁-C₄)-alkyl or N(R14)(R15);
it always being the case that at least one of the radicals R3, R7, R8, R9 and R10 is not hydrogen, and
with the exception of the compound 4-[3-(2,4-dichlorobenzoyl)ureido]-3-methoxybenzoic acid;
and the physiologically tolerated salts thereof.

2. A compound of formula I as claimed in claim 1
wherein
R7, R8, R9 and R10 are, independently of each other, H, F, Cl, Br, OH, NO₂, CN, (C₁-C₆)- alkyl or O-(C₁-C₆)-alkyl, where alkyl can be substituted, once or more than once, by F;
R1 and R2 are H;
R3 is H, F, Cl, Br, NO₂, CN, O-R11, O-phenyl, S-R11, COOR11, N(R12)(R13), (C₁-C₆)-alkyl, (C₂-C₆)- alkenyl, (C₂-C₆)-alkynyl, (C₃-C₇)-cycloalkyl or (C₃-C₇)-cycloalkyl-(C₁-C₄)-alkylene, where alkyl, cycloalkyl and alkynyl can be substituted, once or more than once, by F, Cl, Br, OR11 or COOR11;
R4 is H, F, Cl, NO₂, O-R11, N(R12)(R13) or (C₁-C₆)- alkyl, where alkyl can be substituted, once or more than once, by F;
R5 is H, F, Cl, NO₂, O-R11, N(R12) (R13) or (C₁-C₆)- alkyl, where alkyl can be substituted, once or more than once, by F;
R6 is H, F, Cl, NO₂, O-R11, N(R12)(R13) or (C₁-C₆)- alkyl, where alkyl can be substituted, once or more than once, by F;
R11 is H, (C₁-C₈)-alkyl, (C₁-C₈)-alkylene-O- (C₁-C₈) - alkyl or (C₁-C₈)-alkyl-OH, where alkyl can be substituted, once or more than once, by F;
R12 and R13 are, independently of each other, H or (C₁-C₈)-alkyl;
or R12 and R13 form, together with the nitrogen atom to which they are bonded, a 3-7-membered, saturated heterocyclic ring which can contain up to 2 further heteroatoms from the group N, O or S and where the heterocyclic ring can be substituted, up to four times, by F, Cl, Br, OH, Oxo, (C₁-C₄)-alkyl or N(R14)(R15);
R14 and R15 are, independently of each other, H, (C₁-C₈)-alkyl, (C₂-C₈)-alkenyl, (C₂-C₈)- alkynyl, (C₃-C₇)-cycloalkyl, (C₃-C₇)- cycloalkyl- (C₁-C₄) -alkylene, COO-(C₁-C₄)-alkyl, COO-(C₂-C₄)-alkenyl, phenyl or SO₂-phenyl, where the phenyl ring can be substituted, up to two times, by F, Cl, CN, OH, (C₁-C₆)-alkyl, -(C₁-C₆)-alkyl, CF₃, OCF₃, COOH, COO(C₁-C₆)- alkyl or CONH₂;
with the exception of the compound 4-[3-(2,4-dichlorobenzoyl)ureido]-3-methoxybenzoic acid;
and the physiologically tolerated salts thereof.

3. A compound of formula I as claimed in claim 1 or 2, wherein
R7, R8, R9 and R10 are, independently of each other, H, F, Cl, Br, CH₃ or CF₃;
R1, R2, R5 are H;
R3 is H, F, Cl, NO₂, CF₃, O-R11, N(R12)(R13) or (C₁- C₆)-alkyl, where alkyl can be substituted once or more than once by F;
R4 is H, F, Cl, NO₂, CF₃, O-R11, N(R12)(R13) or (C₁- C₆)-alkyl, where alkyl can be substituted once or more than once by F;
R6 is H, F, Cl; NO₂, CF₃, O-R11, N(R12)(R13) or (C₁- C₆)-alkyl, where alkyl can be substituted once or more than once by F;
R11 is H or (C₁-C₈)-alkyl, where alkyl can be substituted once or more than once by F,
R12 and R13 are H or (C₁-C₈)-alkyl;
or the two radicals R12 and R13 form, together with the
nitrogen atom to which they are bonded, a 5-6-membered, saturated heterocyclic ring which can contain a further oxygen atom;
with the exception of the compound 4-[3-(2,4-dichlorobenzoyl)ureido]-3-methoxybenzoic acid; and the physiologically tolerated salts thereof.

4. A compound of the formula I as claimed in one or more of claims 1 to 3, wherein
R7, R8, R9 and R10 are, independently of each other, H, F, Cl, Br, CH₃ or CF₃;
R1, R2, R4, R5 and R6 are H;
R3 is H, F, Cl, NO₂, O-R11, N(R12)(R13) or (C₁-C₆)- alkyl, where alkyl can be substituted once or more than once by F;
R11 is H or (C₁-C₈)-alkyl, where alkyl can be substituted once or more than once by F,
R12 and R13 are H or (C₁-C₈)-alkyl; or the two radicals R12 and R13 form, together with the nitrogen atom to which they are bonded, a 5- 6-membered, saturated heterocyclic ring which can contain a further oxygen atom;
with the exception of the compound 4-[3-(2,4-dichlorobenzoyl)ureido]-3-methoxybenzoic acid;
and the physiologically tolerated salts thereof.

5. A compound of the formula I as claimed in one or more of claims 1 to 4, wherein it is, in each case, the tromethamol salt.

6. A pharmaceutical which comprises one or more of the compounds as claimed in one or more of claims 1 to 5.

7. A pharmaceutical which comprises one or more of the compounds as claimed in one or more of claims 1 to 5 and one or more blood sugar-lowering active compounds.

8. A pharmaceutical which comprises one or more of the compounds as claimed in one or more of claims 1 to 5 and one or more statins.

9. The use of the compounds as claimed in one or more of claims 1 to 5 for producing a medicament for treating type 2 diabetes.

10. The use of the compounds as claimed in one or more of claims 1 to 5 for producing a medicament for lowering blood sugar.

11. The use of the compounds as claimed in one or more of claims 1 to 5 in combination with at least one further blood sugar-lowering active compound for producing a medicament for treating type 2 diabetes.

12. The use of the compounds as claimed in one or more of claims 1 to 5 in combination with at least one further blood sugar-lowering active compound for producing a medicament for lowering blood sugar.

13. A process for producing a pharmaceutical which comprises one or more of the compounds as claimed in one or more of claims 1 to 5, which comprises mixing the active compound with a pharmaceutically suitable excipient and bringing this mixture into a form which is suitable for administration.

14. A process for preparing the compounds of the formula I, which comprises reacting ureas of the formula 2 or aniline derivatives of the formula 3 with aroyl isocyanates, with reactive acid derivatives, with acid chlorides or with anhydrides, of the formula 4, 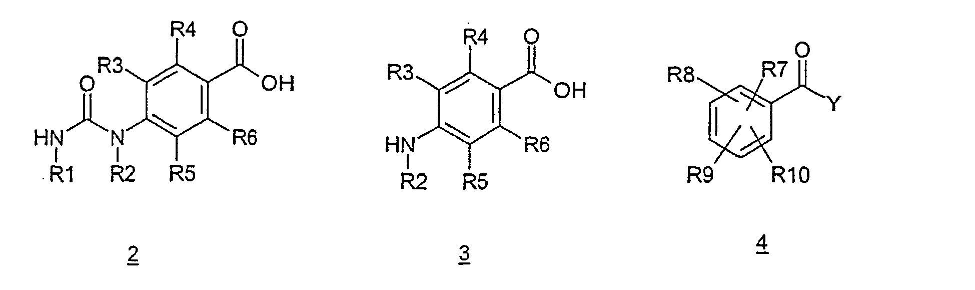 in which R1 to R10 have the meanings given in claim 1.

15. The process for preparing the compounds of the formula I as claimed in claim 14, wherein aroyl isocyanate of the formula 4a is reacted with acid of the formula 3a, 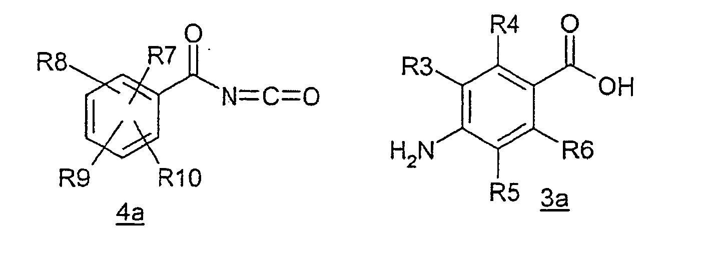 in which R1 to R10 have the meanings given in claim 1.

16. A process for preparing the physiologically tolerated salts of the compounds of the formula I, which comprises reacting the free acids of the compounds of the formula I with a corresponding base.

17. The process as claimed in claim 16, wherein the base is α,α,α-tris(hydroxymethyl)methylamine (tromethamol).

18. A process for preparing the compounds of the formula I, which comprises, in accordance with the following reaction scheme in which R1 to R10 have the meanings given in claim 1,
for example: 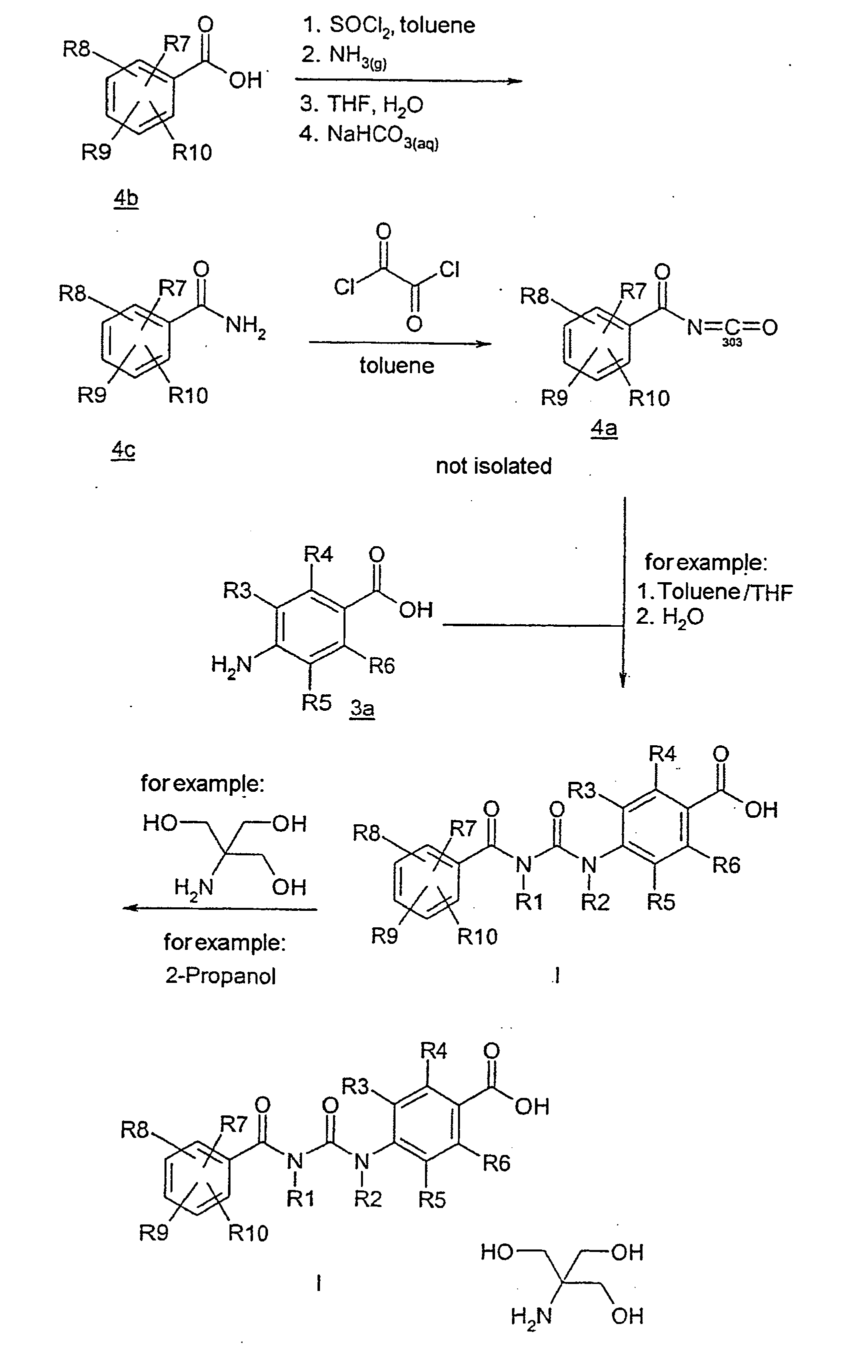 in a first step, using a suitable reagent to convert the compound 4b, in a solvent which is suitable for the purpose, into the acid chloride, and
in a second step, using a suitable reagent to convert the resulting acid chloride, in a solvent which is suitable for the purpose, into the acid amide 4b, and
in a third step, converting the acid amide 4b, by reacting it with oxalyl chloride, into the aroyl isocyanate 4a, and
in a fourth step, reacting the aroyl isocyanate 4a with the aniline 3a, in a suitable solvent, to give the free acid of the compound of the formula I, and
in a fifth step, reacting the free acid of the compound of the formula I with a base to give a physiologically tolerated salt of the compound of the formula 1.

## Revendications

1. Composés de formule I, 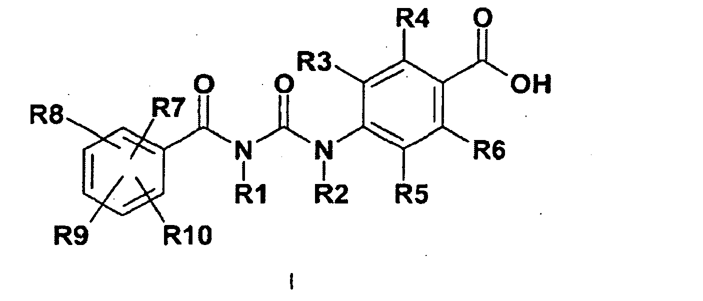 où
R7, R8, R9, R10 signifient, indépendamment l'un de l'autre H, F, Cl, Br, OH, NO₂, CN, O-(C₁- C₆)-alkyle, O-(C₂-C₆)-alcényle, O-(C₂-C₆)-alcynyle, O-SO₂-(C₁-C₄)-alkyle, (C₁-C₆)-alkyle, (C₂-C₆)- alcényle, (C₂-C₆)-alcynyle, où alkyle, alcényle et alcynyle peuvent être monosubstitués ou polysubstitués par F, Cl ou Br ;
R1, R2 signifient, indépendamment l'un de l'autre, H, (C₁-C₆)-alkyle, où alkyle peut être substitué par OH, O-(C₁-C₄)-alkyle, NH₂, NH(C₁-C₄)-alkyle, N[(C₁- C₆)-alkyle]₂, O-(C₁-C₆) -alkyle, CO-(C₁-C₆)-alkyle, COO-(C₁-C₆)-alkyle, (C₁-C₆)-alkylène-COOH ou (C₁- C₆)-alkylène-COO-(C₁-C₆)-alkyle ;
R3 signifie H, F, Cl, Br, NO₂, CN, O-R11, O-phényle, S-R11, COOR11, N(R12)(R13), (C₁-C₆)-alkyle, (C₂-C₆)- alcényle, (C₂-C₆)-alcynyle, (C₃-C₇)-cycloalkyle ou (C₃-C₇)-cycloalkyl-(C₁-C₄)-alkylène, où alkyle, cycloalkyle et alcynyle peuvent être monosubstitués ou polysubstitués par F, Cl, Br, OR11, COOR11 ou N(R16)(R17) ;
R4 signifie H, F, Cl, Br, NO₂, CN, O-R11, O-phényle, S-R11, COOR11, N(R12)(R13), (C₁-C₆)-alkyle, (C₂-C₆)- alcényle, (C₂-C₆) -alcynyle, (C₃-C₇)-cycloalkyle ou (C₃-C₇)-cycloalkyl-(C₁-C₄)-alkylène, où alkyle, cycloalkyle et alcynyle peuvent être monosubstitués ou polysubstitués par F, Cl, Br, OR11, COOR11 ou N(R16)(R17) ;
R5 signifie H, F, Cl, Br, NO₂, CN, O-R11, O-phényle, S-R11, COOR11, N(R12)(R13), (C₁-C₆)-alkyle, (C₂-C₆)- alcényle, (C₂-C₆)-alcynyle, (C₃-C₇)-cycloalkyle ou (C₃-C₇)-cycloalkyl-(C₁-C₄)-alkylène, où alkyle, cycloalkyle et alcynyle peuvent être monosubstitués ou polysubstitués par F, Cl, Br, OR11, COOR11 ou N(R16)(R17) ;
R6 signifie H, F, Cl, Br, NO₂, CN, O-R11, O-phényle, S-R11, COOR11, N(R12)(R13), (C₁-C₆)-alkyle, (C₂-C₆)- alcényle, (C₂-C₆)-alcynyle, (C₃-C₇)-cycloalkyle ou (C₃-C₇)-cycloalkyl-(C₁-C₄)-alkylène, où alkyle, cycloalkyle et alcynyle peuvent être monosubstitués ou polysubstitués par F, Cl, Br, OR11, COOR11 ou N(R16)(R17) ;
R11 signifie H, (C₁-C₈)-alkyle, (C₂-C₈)-alcênyle ou (C₂- C₈)-alcynyle, où alkyle, alcényle et alcynyle peuvent être monosubstitués ou polysubstitués par F, Cl, Br, OH ou O-(C₁-C₄)-alkyle ;
R12, R13 signifient, indépendamment l'un de l'autre H, (C₁-C₈)-alkyle, (C₂-C₈)-alcényle, (C₂-C₈)-alcynyle, (C₃-C₇)-cycloalkyle, (C₃-C₇) -cycloalkyl- (C₁-C₄) - alkylène, COO-(C₁-C₄) -alkyle, COO-(C₂-C₄)-alcényle, phényle ou SO₂-phényle, où le cycle phényle peut être jusqu'à disubstitué par F, Cl, CN, OH, (C₁- C₆)-alkyle, O-(C₁-C₆)-alkyle, CF₃, OCF₃, COOH, COO- (C₁-C₆)-alkyle ou CONH₂ ;
ou R12 et R13 forment ensemble avec l'atome d'azote auquel ils sont liés un cycle hétérocyclique de 3- 7 chaînons, saturé, qui peut contenir jusqu'à 2 autres hétéroatomes du groupe N, O ou S et où le cycle hétérocyclique peut être jusqu'à tétrasubstitué par F, Cl, Br, OH, oxo, (C₁-C₄)- alkyle ou N(R14)(R15) ;
R14, R15 signifient, indépendamment l'un de l'autre H, (C₁-C₈)-alkyle, (C₂-C₈) -alcényle, (C₂-C₈) -alcynyle, (C₃-C₇)-cycloalkyle, (C₃-C₇)-cycloalkyl-(C₁-C₄)- alkylène, COO-(C₁-C₄)-alkyle, COO-(C₂-C₄)-alcényle, phényle ou SO₂-phényle, où le cycle phényle peut être jusqu'à disubstitué par F, Cl, CN, OH, (C₁- C₆)-alkyle, O-(C₁-C₆)-alkyle, CF₃, OCF₃, COOH, COO- (C₁-C₆)-alkyle ou CONH₂ ;
R16, R17 signifient, indépendamment l'un de l'autre H, (C₁-C₈)-alkyle, (C₂-C₈)-alcényle, (C₂-C₈) -alcynyle, (C₃-C₇)-cycloalkyle, (C₃-C₇)-cycloalkyl-(C₁-C₄)- alkylène, COO-(C₁-C₄)-alkyle, COO-(C₂-C₄)-alcényle, phényle ou SO₂ phényle, où le cycle phényle peut être jusqu'à disubstitué par F, Cl, CN, OH, (C₁- C₆)-alkyle, O-(C₁-C₆)-alkyle, CF₃, OCF₃, COOH, COO- (C₁-C₆)-alkyle ou CONH₂ ;
ou R16 et R17 forment ensemble avec l'atome d'azote auquel ils sont liés un cycle hétérocyclique de 3- 7 chaînons, saturé, qui peut contenir jusqu'à 2 autres hétéroatomes du groupe N, O ou S et où le cycle hétérocyclique peut être jusqu'à tétrasubstitué par F, Cl, Br, OH, oxo, (C₁-C₄)- alkyle ou N(R14)(R15) :
- où au moins un des radicaux R3, R7, R8, R9 et R10 est toujours différent d'hydrogène et
- où le composé acide 4-[3-(2,4-dichlorobenzoyl)-uréido]-3-méthoxybenzoïque est exclu ;
ainsi que leurs sels physiologiquement acceptables.

2. Composés de formule I, selon la revendication 1, **caractérisés en ce que**
R7, R8, R9, R10 signifient, indépendamment l'un de l'autre, H, F, Cl, Br, OH, NO₂, CN, (C₁- C₆)-alkyle ou O-(C₁-C₆)-alkyle, où alkyle peut être monosubstitué ou polysubstitué par F ;
R1, R2 signifient H ;
R3 signifie H, F, Cl, Br, NO₂, CN, O-R11, O-phényle, S-R11, COOR11, N(R12)(R13), (C₁-C₆)-alkyle, (C₂-C₆)- alcényle, (C₂-C₆)-alcynyle, (C₃-C₇)-cycloalkyle ou (C₃-C₇)-cycloalkyl-(C₁-C₄)-alkylène, où alkyle, cycloalkyle et alcynyle peuvent être monosubstitués ou polysubstitués par F, Cl, Br, OR11 ou COOR11 ;
R4 signifie H, F, Cl, NO₂, O-R11, N(R12)(R13) ou (C₁- C₆)-alkyle, où alkyle peut être monosubstitué ou polysubstitué par F ;
R5 signifie H, F, Cl, NO₂, O-R11, N(R12)(R13) ou (C₁- C₆)-alkyle, où alkyle peut être monosubstitué ou polysubstitué par F ;
R6 signifie H, F, Cl, NO₂, O-R11, N(R12)(R13) ou (C₁- C₆)-alkyle, où alkyle peut être monosubstitué ou polysubstitué par F ;
R11 signifie H, (C₁-C₈)-alkyle, (C₁-C₈)-alkylène-O-(C₁- C₈)-alkyle ou (C₁-C₈)-alkyl-OH, où alkyle peut être monosubstitué ou polysubstitué par F ;
R12, R13 signifient indépendamment l'un de l'autre H ou (C₁-C₈)-alkyle ;
ou R12 et R13 forment ensemble avec l'atome d'azote auquel ils sont liés un cycle hétérocyclique de 3- 7 chaînons, saturé, qui peut contenir jusqu'à 2 autres hétéroatomes du groupe N, O ou S et où le cycle hétérocyclique peut être jusqu'à tétrasubstitué par F, Cl, Br, OH, oxo, (C₁-C₄)- alkyle ou N(R14)(R15) ;
R14, R15 signifient, indépendamment l'un de l'autre H, (C₁-C₈)-alkyle, (C₂-C₈)-alcényle, (C₂-C₈)-alcynyle, (C₃-C₇)-cycloalkyle, (C₃-C₇)-cycloalkyl-(C₁-C₄)- alkylène, COO-(C₁-C₄)-alkyle, COO-(C₂-C₄)-alcényle, phényle ou SO₂-phényle, où le cycle phényle peut être jusqu'à disubstitué par F, Cl, CN, OH, (C₁- C₆)-alkyle, O-(C₁-C₆)-alkyle, CF₃, OCF₃, COOH, COO- (C₁-C₆)-alkyle ou CONH₂ ;
- où le composé acide 4-[3-(2,4-dichlorobenzoyl)-uréidol-3-méthoxybenzoïque est exclu ;
ainsi que leurs sels physiologiquement acceptables.

3. Composés de formule I, selon la revendication 1 ou 2, **caractérisés en ce que**
R7, R8, R9, R10 signifient indépendamment l'un de l'autre, H, F, Cl, Br, CH₃ ou CF₃ ;
R1, R2, R5 signifient H ;
R3 signifie H, F, Cl, NO₂, CF₃, O-R11, N(R12)(R13) ou (C₁-C₆)-alkyle, où alkyle peut être monosubstitué ou polysubstitué par F ;
R4 signifie H, F, Cl, NO₂, CF₃, O-R11, N(R12)(R13) ou (C₁-C₆)-alkyle, où alkyle peut être monosubstitué ou polysubstitué par F ;
R6 signifie H, F, Cl, NO₂, CF₃, O-R11, N(R12)(R13) ou (C₁-C₆)-alkyle, où alkyle peut être monosubstitué ou polysubstitué par F ;
R11 signifie H ou (C₁-C₈)-alkyle, où alkyle peut être monosubstitué ou polysubstitué par F ;
R12, R13 signifient H ou (C₁-C₈)-alkyle ;
ou les deux radicaux R12 et R13 forment ensemble avec l'atome d'azote auquel ils sont liés un cycle hétérocyclique saturé de 5-6 chaînons, qui peut contenir un autre atome d'oxygène ;
- où le composé acide 4-[3-(2,4-dichlorobenzoyl)-uréido]-3-méthoxybenzoïque est exclu ;
ainsi que leurs sels physiologiquement acceptables.

4. Composés de formule I selon l'une ou plusieurs des revendications 1 à 3, **caractérisés en ce que**
R7, R8, R9, R10 signifient indépendamment l'un de l'autre, H, F, Cl, Br, CH₃ ou CF₃ ;
R1, R2, R4, R5, R6 signifient H ;
R3 signifie H, F, Cl, NO₂, O-R11, N(R12)(R13) ou (C₁- C₆)-alkyle, où alkyle peut être monosubstitué ou polysubstitué par F ;
R11 signifie H ou (C₁-C₈)-alkyle, où alkyle peut être monosubstitué ou polysubstitué par F ;
R12, R13 signifient H ou (C₁-C₈)-alkyle ;
ou les deux radicaux R12 et R13 forment avec l'atome d'azote auquel ils sont liés un cycle hétérocyclique saturé de 5-6 chaînons, qui peut contenir un autre atome d'oxygène ;
- où le composé acide 4-[3-(2,4-dichlorobenzoyl)-uréido]-3-méthoxybenzoïque est exclu ; ainsi que leurs sels physiologiquement acceptables.

5. Composés de formule I selon l'une ou plusieurs des revendications 1 à 4, **caractérisés en ce qu'**il s'agit à chaque fois du sel de Trométamol.

6. Médicament contenant un ou plusieurs des composés selon l'une ou plusieurs des revendications 1 à 5.

7. Médicament contenant un ou plusieurs des composés selon l'une ou plusieurs des revendications 1 à 5 et une ou plusieurs substances actives diminuant la glycémie.

8. Médicament contenant un ou plusieurs des composés selon l'une ou plusieurs des revendications 1 à 5 et une ou plusieurs statines.

9. Utilisation des composés selon l'une ou plusieurs des revendications 1 à 5 pour la préparation d'un médicament destiné au traitement du diabète de type 2.

10. Utilisation des composés selon l'une ou plusieurs des revendications 1 à 5 pour la préparation d'un médicament destiné à abaisser la glycémie.

11. Utilisation des composés selon l'une ou plusieurs des revendications 1 à 5 en combinaison avec au moins une autre substance active diminuant la glycémie pour la préparation d'un médicament destiné au traitement du diabète de type 2.

12. Utilisation des composés selon l'une ou plusieurs des revendications 1 à 5 en combinaison avec au moins une autre substance active diminuant la glycémie pour la préparation d'un médicament destiné à la diminution de la glycémie.

13. Procédé pour la préparation d'un médicament contenant un ou plusieurs des composés selon l'une ou plusieurs des revendications 1 à 5, **caractérisé en ce que** la substance active est mélangée avec un support pharmaceutiquement approprié et ce mélange est amené dans une forme appropriée pour l'administration.

14. Procédé pour la préparation des composés de formule I, **caractérisé en ce qu'**on transforme des urées de formule 2 ou des dérivés d'aniline de formule 3 avec des aroylisocyanates, avec des dérivés d'acide réactifs, avec des chlorures d'acide ou avec des anhydrides, de formule 4, 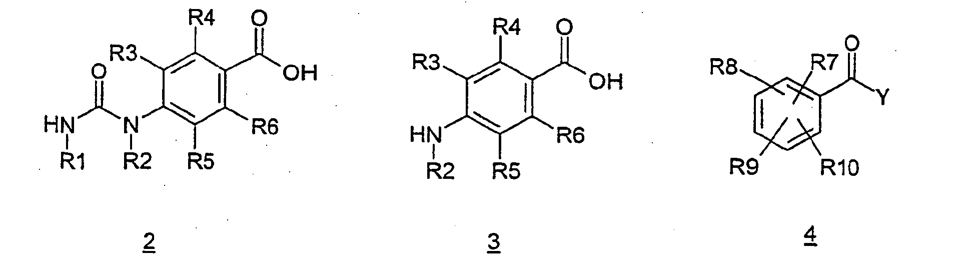 où R1 à R10 ont les significations indiquées dans la revendication 1.

15. Procédé pour la préparation des composés de formule I selon la revendication 14, **caractérisé en ce que** l'aroylisocyanate de formule 4a est transformé avec un acide de formule 3a, 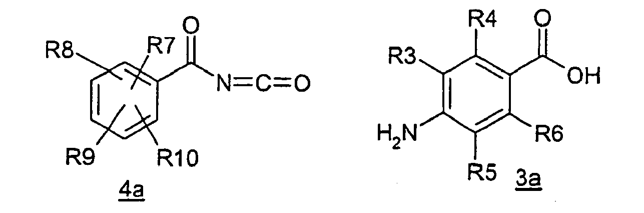 où R1 à R10 ont les significations indiquées dans la revendication 1.

16. Procédé pour la préparation des sels physiologiquement acceptables des composés de formule I, **caractérisé en ce que** les acides libres des composés de formule I sont transformés avec une base correspondante.

17. Procédé selon la revendication 16, **caractérisé en ce que** la base est l'α,α,α-tris-(hydroxyméthyl)-méthylamine (Trométhamol).

18. Procédé pour la préparation des composés de formule I, **caractérisé en ce que**, selon le schéma de réactions suivant, où R1 à R10 ont les significations indiquées dans la revendication 1, 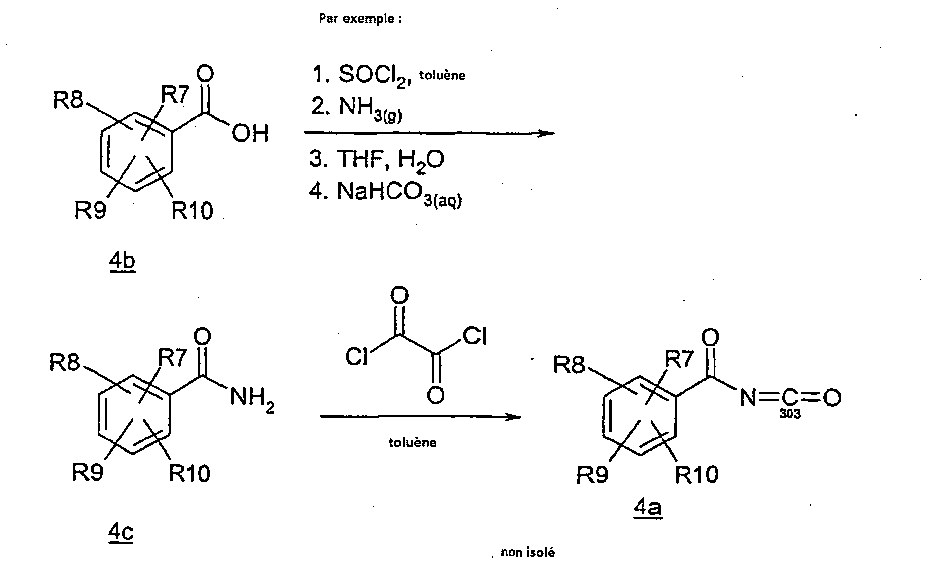 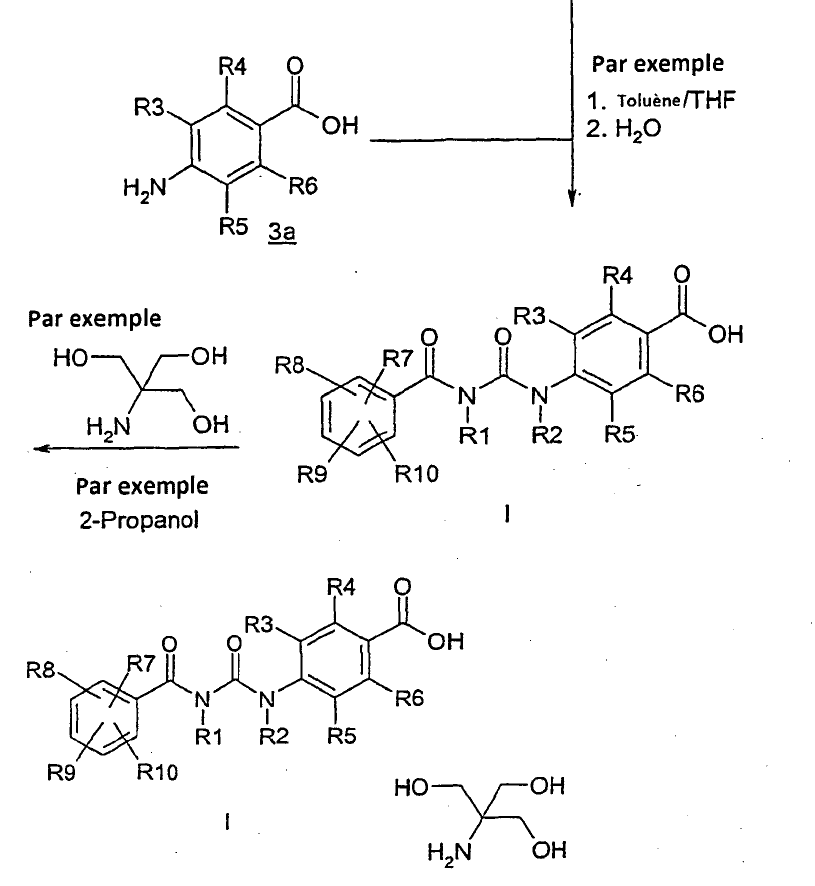
- dans une première étape, on transforme le composé 4b, dans un solvant approprié à cet effet, au moyen d'un réactif approprié, en chlorure d'acide et
- dans une deuxième étape, on transforme le chlorure d'acide ainsi obtenu, dans un solvant approprié à cet effet, au moyen d'un réactif approprié, en amide d'acide 4b et
- dans une troisième étape, on transforme l'amide d'acide 4b par transformation avec du chlorure d'oxalyle en aroylisocyanate 4a et
- dans une quatrième étape, on transforme l'aroylisocyanate 4a avec l'aniline 3a dans un solvant approprié en acide libre du composé de formule I et
- dans une cinquième étape, on transforme l'acide libre du composé de formule I avec une base en un sel physiologiquement acceptable du composé de formule I.
